# FASCICULE DE BREVET EUROPEEN

(11) **EP 1 453 597 B1**
(45) Date de publication et mention de la délivrance du brevet: **13.08.2008**
(21) Numéro de dépôt: 02801121.1
(22) Date de dépôt: 12.12.2002
(51) Int. Cl.: B01F 17/00

(54) **UTILISATION DE COPOLYMERES CATIONIQUES A BLOCS COMME AIDE AU DEPOT D'EMULSIONS SIMPLES OU MULTIPLES**
VERWENDUNG VON KATIONISCHEN BLOCKCOPOLYMEREN ALS BESCHICHTIGUNGSHILFSMITTEL VON EINFACHEN UND MULTIPLEN EMULSIONEN
USE OF CATIONIC BLOCK POLYMERS TO ASSIST DEPOSITION OF SINGLE OR MULTIPLE EMULSIONS

(30) Priorité: 12.12.2001 FR 0116234
(43) Date de publication de la demande: 08.09.2004
(73) Titulaire: RHODIA CHIMIE, 92512 Boulogne Billancourt Cedex (FR)
(72) Inventeur: BAVOUZET, Bruno, F-75010 Paris (FR); DESTARAC, Mathias, F-75005 Paris (FR); WILCZEWSKA, Agnieszka, 15-078 Bialystok (PL)
(74) Mandataire: Boittiaux, Vincent
(86) Numéro de dépôt international: PCT/FR2002/004319
(87) Numéro de publication internationale: WO 2003/049848

(56) Documents cités:
- EP-A- 0 345 075
- EP-A- 0 631 774
- US-A- 5 656 263
- LUCA DE M ET AL: "A Stable w/o/w Multiple Emulsion" COSMETICS & TOILETRIES, WHEATON, IL, US, vol. 105, novembre 1990 (1990-11), pages 65-67, XP002215250 ISSN: 0361-4387

## Description

La présente invention a pour objet l'utilisation comme aide au dépôt d'une émulsion sur une surface, d'au moins un copolymère à blocs comprenant au moins un bloc hydrophobe non ionique et au moins un bloc à motifs cationiques dans les conditions d'utilisation de ladite émulsion.

Dans certaines applications, que ce soit dans le domaine cosmétique, le domaine de la détergence, le domaine phytosanitaire, ou encore celui du traitement du métal, on cherche à modifier l'état de diverses surfaces par le dépôt de compositions au moyen de formulations aqueuses, qui sont pour la plupart des cas, des émulsions.

La présente invention a trait à ce domaine particulier, et plus spécialement, l'un des objets de l'invention est l'utilisation de certains copolymères à blocs comme aide au dépôt d'émulsions.

Ainsi, l'invention a donc pour objet l'utilisation comme aide au dépôt d'une émulsion sur une surface, d'au moins un copolymère à blocs comprenant au moins un bloc hydrophobe non ionique et au moins un bloc à motifs cationiques dans les conditions d'utilisation de ladite émulsion ; ladite émulsion comprenant une phase organique dispersée dans une phase aqueuse et comprenant éventuellement au moins un tensioactif et/ou au moins un polymère amphiphile externe(s).

Un autre objet de l'invention consiste en un procédé pour aider le dépôt d'une émulsion sur une surface, dans les conditions d'utilisation de ladite émulsion, par addition à ladite émulsion d'au moins un copolymère à blocs comprenant au moins un bloc hydrophobe non ionique et au moins un bloc à motifs cationiques dans les conditions d'utilisation de ladite émulsion ; ladite émulsion comprenant une phase organique dispersée dans une phase aqueuse et comprenant éventuellement au moins un tensioactif et/ou au moins un polymère amphiphile externe(s).

D'autres avantages et caractéristiques de la présente invention apparaîtront plus clairement à la lecture de la description qui va suivre.

Avant tout, il est indiqué que la présente invention est adaptée pour des émulsions simples directes, c'est-à-dire comprenant une phase organique dispersée dans une phase aqueuse.

Elle est aussi appropriée pour des émulsions multiples. Selon une première variante, l'émulsion multiple comprend une phase aqueuse interne dispersée dans la phase organique, l'ensemble phase aqueuse interne et phase organique étant dispersé dans la phase aqueuse. Selon une deuxième variante, l'émulsion multiple comprend une phase organique interne dispersée dans la phase organique, l'ensemble phase organique interne et phase organique étant dispersé dans la phase aqueuse.

Dans la suite de la description, les termes "phase aqueuse" désignent la phase aqueuse d'une émulsion simple ou de la phase aqueuse externe d'une émulsion multiple ; les termes "phase organique" désignent la phase organique d'une émulsion simple ou la phase organique en contact avec la phase aqueuse externe d'une émulsion multiple; les termes "phase aqueuse interne" désignent la phase aqueuse interne d'une émulsion multiple ; les termes "phase organique interne" désignent la phase organique interne d'une émulsion multiple.

Par ailleurs, lorsqu'il sera dit d'un (co)polymère ou d'un tensioactif qu'il remplit les conditions de la règle de Bancroft, cela signifiera que la fraction du (co)polymère ou du tensioactif qui est soluble dans la phase continue de l'émulsion est supérieure à la fraction qui est soluble dans la phase dispersée de l'émulsion ; que l'on considère les deux phases d'une émulsion simple, ou les phases d'une émulsion multiple, deux à deux, (soit la phase interne et la phase en contact avec la phase aqueuse externe, ou la phase en contact avec la phase aqueuse externe et la phase aqueuse externe).

Plus particulièrement, le (co)polymère ou le tensioactif est choisi parmi ceux qui vérifient à la fois les deux conditions ci-dessous :
- lorsqu'il est mélangé avec la phase aqueuse, à une concentration comprise entre 0,1 et 10 % en poids de ladite phase à 25°C, se trouve sous la forme d'une solution dans tout ou partie de la gamme de concentration indiquée ;
- lorsqu'il est mélangé avec la phase organique, à une concentration comprise entre 0,1 et 10 % en poids de ladite phase et à 25°C, se trouve sous la forme d'une dispersion dans tout ou partie de la gamme de concentration indiquée.

Le copolymère à blocs va tout d'abord être décrit.

Ainsi le copolymère à blocs mis en oeuvre dans le cadre de la présente invention peut se présenter sous une forme linéaire (multiblocs), sous une forme ramifiée (peigne ou greffée), ou encore sous une forme étoile.

Les copolymères à blocs linéaires ont plus particulièrement une structure comprenant deux blocs (diblocs).

Les copolymères à blocs de structure ramifiée (peigne ou greffé) présentent, de manière préférentielle, un squelette non ionique, de préférence hydrophile, comprenant des segments pendants cationiques et hydrophobes non ioniques.

En ce qui concerne les copolymères à blocs de structure étoile, plusieurs possibilités sont envisageables. Selon un mode de réalisation particulier, si l'on considère chaque branche de l'étoile, celle-ci peut comprendre soit un copolymère à blocs, de préférence un dibloc, dont l'un des blocs est hydrophobe non ionique, l'autre cationique ; soit un bloc hydrophobe non ionique ou un bloc cationique.

Il est précisé que chaque bloc du copolymère peut être constitué d'un homopolymère ou d'un copolymère statistique ou à blocs ou encore présenter un gradient de concentration des monomères le long de la chaîne du bloc concerné.

Par ailleurs, le copolymère à blocs mis en oeuvre ne peut pas être considéré comme un tensioactif. En effet, la masse molaire en poids du copolymère à blocs est d'au moins 2000g/mol.

Selon une variante avantageuse de l'invention, le copolymère à blocs mis en oeuvre est choisi parmi ceux qui lorsqu'ils sont mélangés à une phase aqueuse, à une concentration comprise entre 0,1 et 10 % en poids de ladite phase à 25°C, se trouvent sous la forme d'une solution dans tout ou partie de la gamme de concentration indiquée.

En outre, le ou les blocs hydrophobes non ioniques du copolymère à blocs sont plus particulièrement obtenus à partir d'au moins un monomère choisi parmi :
- les esters des acides mono- ou poly- carboxyliques, linéaires, ramifiés, cycliques ou aromatiques, comprenant au moins une insaturation éthylénique ;
- les esters d'acides carboxyliques saturés comprenant 8 à 30 atomes de carbone, éventuellement porteurs d'un groupement hydroxyle ;
- les nitriles αβ-éthyléniquement insaturés, les éthers vinyliques, les esters vinyliques, les monomères vinylaromatiques, les halogénures de vinyle ou de vinylidène ;
- les monomères hydrocarbonés, linéaires ou ramifiés, aromatiques ou non, comprenant au moins une insaturation éthylénique ;
- l'oxyde de propylène, l'oxyde de butylène ;
seuls ou en mélanges, ainsi que les macromonomères dérivant de tels monomères.

Il est rappelé que le terme macromonomère désigne une macromolécule portant une ou plusieurs fonctions polymérisables par la méthode choisie de polymérisation.

Il est précisé de plus que les blocs hydrophobes du copolymère à blocs sont obtenus à partir de monomères exempts d'atomes de silicium.

A titre d'exemples particuliers de monomères utilisables dans la préparation du ou des blocs hydrophobes des copolymères à blocs, on peut citer :
- les esters d'acide (méth)acrylique avec un alcool comprenant 1 à 12 atomes de carbone comme le (méth)acrylate de méthyle, le (méth)acrylate d'éthyle, le (méth)acrylate de propyle, le (méth)acrylate de n-butyle, le (méth)acrylate de t-butyle, le (méth)acrylate d'isobutyle, l'acrylate de 2-éthylhexyl, l'acrylate d'isodécyle ;
- l'acétate de vinyle, le Versatate^{®} de vinyle, le propionate de vinyle, le chlorure de vinyle, le chlorure de vinylidène, le méthyl vinyléther, l'éthyl vinyléther ;
- les nitriles vinyliques incluent plus particulièrement ceux ayant de 3 à 12 atomes de carbone, comme en particulier l'acrylonitrile et le méthacrylonitrile ;
- le styrène, l'α-méthylstyrène, le vinyltoluène, le butadiène, le chloroprène ;
seuls ou en mélanges, ainsi que les macromonomères dérivant de tels monomères.

Les monomères préférés sont les esters de l'acide acryliques avec les alcools linéaires ou ramifiés en C₁-C₄ tels que le (méth)acrylate de méthyle, d'éthyle, de propyle et de butyle, les esters vinyliques comme l'acétate de vinyle, le styrène, l'α-méthylstyrène.

Ajoutons que selon un mode de réalisation avantageux de l'invention, les monomères hydrophobes sont choisis de telle sorte que le polymère correspondant au bloc hydrophobe (à masse molaire en poids similaire à celle du bloc) présente une température de transition vitreuse, Tg, inférieure ou égale à 50°C.

Quant au(x) bloc(s) à motifs cationique(s) du copolymère à blocs, ces derniers peuvent avantageusement être obtenus à partir d'au moins un monomère choisi parmi :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, aromatique ou non, la vinylamine, l'éthylène imine ;
- les sels d'ammonium de diallyldialkyl ;
seuls ou en mélanges, ou les sels correspondants, ainsi que les macromonomères dérivant de tels monomères.

A titre d'exemples plus précis de monomères cationiques à partir desquels le copolymère à blocs peut être synthétisé, on peut citer notamment :
- diméthyl amino éthyl (méth)acrylate, diméthyl amino propyl (méth)acrylate le ditertiobutyl aminoéthyl (méth)acrylate, le diméthyl amino méthyl (méth)acrylamide, le diméthyl amino propyl (méth)acrylamide, le chlorure de triméthylammonium éthyl (méth)acrylate, le méthyl sulfate de triméthylammonium éthyl acrylate, le chlorure de benzyl diméthylammonium éthyl (méth)acrylate, le chlorure de 4-benzoylbenzyl diméthyl ammonium éthyl acrylate, le chlorure de triméthyl ammonium éthyl (méth)acrylamido ;
- l'éthylène imine, la vinylamine, la pyridine, la 2-vinylpyridine, la 4-vinylpyridine, la pyrrolidine, la vinyl-1 pyrrolidine-2, l'imidazoline, la vinyl-1 imidazoline-3, la vinylamine, l'éthylène imine ;
- le chlorure de triméthyl ammonium de vinylbenzyle ;
- le chlorure d'ammonium de diallyldiméthyl ;
seuls ou en mélanges, ou leurs sels correspondants, ainsi que les macromonomères dérivant de tels monomères.

Lorsque les monomères se trouvent sous forme de sels, et plus particulièrement avec des fonctions amines quaternisées, de type ammonium NR₄+ avec R, identiques ou non, représentant un atome d'hydrogène, un radical alkyle ou hydroxyalkyle comprenant 1 à 10 atomes de carbone, de préférence 1 à 4, éventuellement porteur d'un radical hydroxyle ; le contre-ion peut être choisi parmi les halogénures comme par exemple le chlore, les sulfates, les hydrosulfates, les alkylsulfates (par exemple comprenant 1 à 6 atomes de carbone), les phosphates, les citrates, les formates, les acétates.

Il est précisé que le ou les blocs cationiques du copolymère à blocs peuvent se trouver sous une forme non cationique dans l'émulsion, dès l'instant que dans les conditions d'utilisation de ladite émulsion, lesdits blocs se trouvent sous une forme cationique.

Il n'est pas exclu que l'un et/ou l'autre des deux blocs comprennent un ou plusieurs monomères hydrophiles non ioniques.

A titre d'exemples de monomères de ce type, on peut citer entre autres, seuls ou en mélange, ou sous la forme de macromonomères, l'oxyde d'éthylène ; les amides des acides mono- ou poly- carboxyliques, linéaires, ramifiés, cycliques ou aromatiques, comprenant au moins une insaturation éthylénique ou dérivés, comme le (méth)acrylamide, le N-méthylol (méth)acrylamide ; les esters hydrophiles dérivant de l'acide (méth)acrylique comme par exemple le (méth)acrylate de 2-hydroxyéthyle ; les esters vinyliques permettant d'obtenir des blocs alcool polyvinylique après hydrolyse, comme l'acétate de vinyle, le Versatate^{®} de vinyle, le propionate de vinyle ; les monomères du type des sucres comme les osides, les polyholosides fortement dépolymérisés.

Par fortement dépolymérisés, on entend des composés dont la masse moléculaire en poids est plus particulièrement inférieure à 20000 g/mole.

Les osides sont des composés qui résultent de la condensation, avec élimination d'eau, de molécules d'oses entre elles ou encore de molécules d'oses avec des molécules non glucidiques. Parmi les osides on préfère les holosides qui sont formés par la réunion de motifs exclusivement glucidiques et plus particulièrement les oligoholosides (ou oligosaccharides) qui ne comportent qu'un nombre restreint de ces motifs, c'est-à-dire un nombre en général inférieur ou égal à 10. A titre d'exemples d'oligoholosides on peut mentionner le saccharose, le lactose, la cellobiose, le maltose.

Les polyholosides (ou polysaccharides) fortement dépolymérisés convenables sont décrits par exemple dans l'ouvrage de P. ARNAUD intitulé "cours de chimie organique", GAUTHIER-VILLARS éditeurs, 1987. A titre d'exemple non limitatif de polyholosides fortement dépolymérisés, on peut citer le dextran, l'amidon.

Les proportions respectives des divers types de monomères qu'ils soient cationiques, hydrophobes non ioniques, et le cas échant hydrophiles non ioniques, sont telles que le copolymère à blocs résultant satisfasse la règle de Bancroft.

De manière particulière, la masse molaire en poids du copolymère à blocs est comprise entre 2000 et 100000 g/mol, de préférence entre 2000 et 20000 g/mol. Cette masse est une masse absolue, déterminée par chromatographie par exclusion stérique couplée à la méthode MALLS.

De plus, et selon une variante de l'invention, le copolymère à blocs est tel que la masse molaire en poids des blocs cationiques représente au moins deux fois la masse des blocs hydrophobes non ioniques, de préférence au moins cinq fois.

Conformément à un mode de réalisation préféré de l'invention, le ou les blocs cationiques sont tels qu'au moins 5 % en moles des monomères à partir desquels le(s) bloc(s) sont obtenus (nombre de moles de monomères engagés dans la synthèse du bloc concerné) sont des monomères cationiques, de préférence au moins 30 % en moles. De manière préférée, le ou les blocs cationiques sont obtenus à partir de monomères cationiques, et plus préférentiellement encore, les blocs sont des homoplymères.

Enfin, la teneur en copolymère à blocs dans l'émulsion représente 0,1 à 30 % en poids par rapport au poids de phase organique.

Ces polymères peuvent être obtenus par toute méthode connue, que ce soit par polymérisation radicalaire, contrôlée ou non, par polymérisation par ouverture de cycle (notamment anionique ou cationique), par polymérisation anionique ou cationique, ou encore par modification chimique d'un polymère.

De manière préférée, on met en oeuvre des méthodes de polymérisation radicalaire dite vivante ou contrôlée. A titre d'exemples non limitatifs de procédés de polymérisation dite vivante ou contrôlée, on peut notamment se référer aux demandes WO 98/58974 (xanthate), WO 97/01478 (dithioesters), WO 99/03894 (nitroxydes) ; WO 99/31144 (dithiocarbamates).

Les polymères à blocs greffés ou peignes peuvent être obtenus par des méthodes dites de greffage direct et copolymérisation.

Le greffage direct consiste à polymériser le(s) monomère(s) choisi(s) par voie radicalaire, en présence du polymère sélectionné pour former le squelette du produit final. Si le couple monomère / squelette ainsi que les conditions opératoires, sont judicieusement choisis, alors il peut y avoir réaction de transfert entre le macroradical en croissance et le squelette. Cette réaction génère un radical sur le squelette et c'est à partir de ce radical que croît le greffon. Le radical primaire issu de l'amorceur peut également contribuer aux réactions de transfert.

Pour ce qui a trait à la copolymérisation, elle met en oeuvre dans un premier temps le greffage à l'extrémité du futur segment pendant, d'une fonction polymérisable par voie radicalaire. Ce greffage peut être réalisé par des méthodes usuelles de chimie organique. Puis, dans un second temps, le macromonomère ainsi obtenu est polymérisé avec le monomère choisi pour former le squelette et on obtient un polymère dit "peigne".

Dans le cas de polymères de type étoile, les synthèses peuvent être essentiellement classées en deux groupes. Le premier correspond à la formation des bras des polymères à partir d'un composé plurifonctionnel constituant le centre (technique "core-first") (Kennedy, J.P. and coll. Macromolecules, 29, 8631 (1996), Deffieux, A. and coll. Ibid, 25, 6744, (1992), Gnanou, Y. and coll. Ibid, 31, 6748 (1998)) et le second correspond à une méthode où les molécules de polymères qui vont constituer les bras sont d'abord synthétisées et ensuite liées ensemble sur un coeur pour former un polymère en forme d'étoile (technique "arm-first"). Parmi les méthodes utilisables pour lier les bras, on peut notamment citer la méthode comprenant la réaction de ces bras avec un composé présentant une pluralité de groupes fonctionnels capables de réagir avec des groupements fonctionnels antagonistes terminaux desdits bras (Fetters, L.J. and coll. Macromolecules, 19, 215 (1986), Hadjichristidis, N. and coll. Macromolecules, 26, 2479 (1993), Roovers, J. and coll. Macromolecules, 26, 4324 (1993)). Citons également la méthode comprenant l'ajout d'un composé présentant une pluralité de groupes polymérisables, suivi de la polymérisation desdits bras (Rempp, P. and coll., Polym. Sci. Part C, 22, 145 (1968), Fetters, L J. and coll. Macromolecules, 8, 90 (1975), Higashimura and coll. Ibid, 24, 2309 (1991)).

Pour obtenir les chaînes polymères constituant ultérieurement les bras des étoiles, on a généralement recours à des méthodes permettant de contrôler la réaction de polymérisation. Ainsi, les polymérisations anionique et cationique vivante sont les méthodes les plus utilisées actuellement.

Conformément à un premier mode de réalisation de l'invention, le copolymère à blocs qui vient d'être décrit est utilisé comme aide au dépôt d'une émulsion simple comprenant une phase organique dispersée dans une phase aqueuse.

Le composé utilisé en tant que phase organique est de préférence choisi parmi les composés dont la solubilité dans l'eau ne dépasse pas 10 % en poids, dans une gamme de température comprise entre 20°C et la température de préparation de l'émulsion.

En tant que composé convenable, on peut citer les composés organiques ou organosiliciques, notamment les huiles organiques, d'origine animale ou végétale, ou minérales, ainsi que les cires provenant des mêmes origines, ou leurs mélanges, les huiles, cires ou résines en polyorganosiloxane linéaire, cyclique, ramifié ou réticulé.

Comme huiles organiques d'origine animale, on peut citer entre autres, l'huile de cachalot, l'huile de baleine, l'huile de phoque, l'huile de sardine, l'huile de hareng, l'huile de squale, l'huile de foie de morue ; les graisses de porc, de mouton (suifs).

En tant que cires d'origine animale, on peut citer la cire d'abeille.

A titres d'exemples d'huiles organiques d'origine végétale, on peut mentionner, entre autres, l'huile de colza, l'huile de tournesol, l'huile d'arachide, l'huile d'olive, l'huile de noix, l'huile de maïs, l'huile de soja, l'huile de lin, l'huile de chanvre, l'huile de pépins de raisin, l'huile de coprah, l'huile de palme, l'huile de graines de coton, l'huile de babassu, l'huile de jojoba, l'huile de sésame, l'huile de ricin, le beurre de cacao, le beurre de karité.

En tant que cires d'origine végétale, on peut citer la cire de carnauba.

En ce qui concerne les huiles minérales, on peut citer entre autres les coupes pétrolières, les huiles naphténiques, paraffiniques (vaseline). Les cires paraffiniques peuvent de même convenir à la préparation de l'émulsion.

Les produits issus de l'alcoolyse des huiles précitées peuvent aussi être utilisés.

On ne sortirait du cadre de la présente invention en mettant en oeuvre, en tant que phase organique, au moins un acide gras, saturé ou non, au moins un alcool gras, saturé ou non, au moins un ester d'acide gras, ou leurs mélanges.

Plus particulièrement, lesdits acides comprennent 8 à 40 atomes de carbone, plus particulièrement 10 à 40 atomes de carbone, de préférence 18 à 40 atomes de carbone, et peuvent comprendre une ou plusieurs insaturations éthyléniques, conjuguées ou non, et éventuellement un ou plusieurs groupements hydroxyles. Quant aux alcools, ils peuvent comprendre un ou plusieurs groupements hydroxyles.

Comme exemples d'acides gras saturés, on peut citer les acides palmitique, stéarique, béhénique.

Comme exemples d'acides gras insaturés, on peut citer les acides myristoléique, palmitoléique, oléique, érucique, linoléique, linolénique, arachidonique, ricinoléique, ainsi que leurs mélanges.

Quant aux alcools, ceux-ci comprennent plus particulièrement 4 à 40 atomes de carbone, de préférence 10 à 40 atomes de carbone, éventuellement une ou plusieurs insaturations éthyléniques, conjuguées ou non, et éventuellement plusieurs groupements hydroxyles. Les polymères comprenant plusieurs groupement hydroxyles peuvent de même convenir, comme par exemple les polypropylèneglycols.

Comme exemple d'alcools, on peut citer par exemple ceux correspondants aux acides précités.

Concernant les esters d'acides gras, ceux-ci peuvent avantageusement être obtenus à partir d'acides gras, choisis parmi les composés nommés ci-dessus. Les alcools à partir desquels ces esters sont préparés comprennent plus particulièrement 1 à 6 atomes de carbone. De préférence, il s'agit d'esters méthylique, éthylique, propylique, isopropylique.

Par ailleurs, il n'est pas exclu d'utiliser en tant que phase organique des mono-, di- et tri- glycérides.

Elle peut aussi être choisie parmi les huiles silicones, qui de manière avantageuse sont constituées en tout ou partie de motifs de formule :
R'₃₋ₐRₐSiO_{1/2} (motif M) et/ou R₂SiO (motif D)
formules où :
- a est un entier de 0 à 3 ;
- les radicaux R sont identiques ou différents et représentent :
   - un groupe hydrocarboné aliphatique saturé ou insaturé contenant de 1 à 10 atomes de carbone ;
   - un groupe hydrocarboné aromatique contenant de 6 à 13 atomes de carbone ;
   - un groupe organique polaire lié au silicium par une liaison Si-C ou Si-O-C ;
- les radicaux R' sont identiques ou différents et représentent :
   - un groupe hydrocarboné aliphatique saturé ou insaturé contenant de 1 à 10 atomes de carbone ;
   - un groupe hydrocarboné aromatique contenant de 6 à 13 atomes de carbone ;
   - une fonction -OH ;
   - un groupe amino- ou amido-fonctionnel contenant de 1 à 6 atomes de carbone, lié au silicium par une liaison Si-N.

De préférence au moins 80% des radicaux R représentent un groupe méthyle.

Ces silicones peuvent éventuellement comprendre, de préférence moins de 5 % molaire, des motifs de formules T et/ou Q :
RSiO_{3/2} (motif T) et/ou SiO₂ (motif Q)
formule dans laquelle R a la définition donnée ci-dessus.

A titre d'exemples de radicaux hydrocarbonés aliphatiques ou aromatiques R, on peut citer les groupes :
- alkyle, de préférence alkyle en C₁-C₁₀ éventuellement halogéné, tels que méthyle, éthyle, octyle, trifluoropropyle ;
- alcoxyalkylène, plus particulièrement en C₂-C₁₀, de préférence en C₂-C₆, tels que -CH₂-CH₂-O-CH₃;
- alcényles, de préférence alcényle en C₂-C₁₀, tels que vinyle, allyle, hexényle, décényle, décadiényle ;
- alcényloxyalkylène tels que -(CH₂)₃-O-CH₂-CH=CH₂, ou alcényloxyalcoxy alkyle tels que -(CH₂)₃-OCH₂-CH₂-O-CH=CH₂ dans lesquels les parties alkyle sont de préférence en C₁-C₁₀ et les parties alcényles sont de préférence en C₂-C₁₀;
- aryles, de préférence en C₆-C₁₃, tels que phényle.

A titre d'exemples de groupes organiques polaires R, on peut citer les groupes :
- hydroxyfonctionnels tels que des groupes alkyle substitués par un ou plusieurs groupes hydroxy ou di(hydroxyalkyl)amino et éventuellement interrompu par un ou plusieurs groupes bivalents hydroxyalkylamino. Par alkyle on entend une chaîne hydrocarbonée de préférence en C₁-C₁₀, mieux encore en C₁-C₆; des exemples de ces groupes sont -(CH₂)₃-OH ; - (CH₂)₄N(CH₂CH₂OH)₂ ; -(CH₂)₃-N(CH₂CH₂OH)-CH₂-CH₂-N(CH₂CH₂OH)₂ :
- aminofonctionnels tels que alkyle substitué par un ou plusieurs groupes amino ou aminoalkylamino où alkyle est tel que défini ci-dessus ; des exemples en sont -(CH₂)₃-NH₂ ; (CH₂)₃-NH-(CH₂)₂NH₂ ;
- amidofonctionnels tels que alkyle substitué par un ou plusieurs groupes acylamino et éventuellement interrompu par un ou plusieurs groupes bivalents alkyl-CO-N< où alkyle est tel que défini ci-dessus et acyle représente alkylcarbonyle ; un exemple est le groupe -(CH₂)₃-N(COCH₃)-(CH₂)₂NH(COCH₃) ;
- carboxyfonctionnels tels que carboxyalkyle éventuellement interrompu par un ou plusieurs atomes d'oxygène ou de soufre où alkyle est tel que défini ci-dessus ; un exemple est le groupe -CH₂-CH₂-S-CH₂-COOH.

A titre d'exemples de radicaux R', on peut citer les groupes :
- alkyle, de préférence alkyle en C₁-C₁₀ éventuellement halogéné, tels que méthyle, éthyle, octyle, trifluoropropyle ;
- aryles, de préférence en C₆-C₁₃, tels que phényle ;
- aminofonctionnels tels que alkyle ou aryle substitué par amino, alkyle étant de préférence en C₁-C₆ et aryle désignant un groupe aromatique cyclique hydrocarboné de préférence en C₆-C₁₃ tel que phényle ; des exemples en sont éthylamino, phénylamino ;
- amidofonctionnels tels que alkylcarbonylamino où alkyle est de préférence en C₁-C₆; des exemples en sont méthylacétamido.

A titre d'exemples concrets de "motifs D" on peut citer : (CH₃)₂SiO; CH₃(CH=CH₂)SiO ; CH₃(C₆H₅)SiO ; (C₆H₅)₂SiO ; CH₃(CH₂-CH₂-CH₂-OH)SiO.

A titre d'exemples concrets de "motifs M", on peut citer : (CH₃)₃SiO_{1/2} ; (CH₃)₂(OH)SiO_{1/2} ; (CH₃)₂(CH=CH₂)SiO_{1/2} ; (OCH₃)₃SiO_{1/2} : [O-C(CH₃)=CH₂]₃SiO_{1/2} : [ON=C(CH₃)]₃SiO_{1/2} ; (NH-CH₃)₃SiO_{1/2}; (NH-CO-CH₃)₃SiO_{1/2}.

A titre d'exemples concrets de "motifs T", on peut citer : CH₃SiO_{3/2}; (CH=CH₂)SiO_{3/2}.

Lorsque les silicones contiennent des radicaux R réactifs et/ou polaires (tels que OH, vinyle, allyle, héxényle, aminoalkyles ....), ces derniers ne représentent généralement pas plus de 5% du poids de la silicone, et de préférence pas plus de 1 % du poids de la silicone.

Peuvent être utilisés de préférence des huiles volatiles comme l'hexaméthyldisiloxane, l'octaméthyldisiloxane, le décaméthyltétrasiloxane, le dodécaméthylpentasiloxane, le tétradécaméthylhexasiloxane, l'hexadécaméthylhexa siloxane ; l'heptaméthyl-3[(triméthyl-silyl)oxy]trisiloxane, l'hexaméthyl-3,3 bis[(triméthylsilyl)oxy]trisiloxane ; l'hexaméthylcyclotrisiloxane, l'octaméthylcyclotétrasiloxane, le décaméthylcyclopentasiloxane, le dodécaméthylcyclohexasiloxane, le pentaméthyl[(triméthylsilyl)oxy]cyclotrisiloxane.

On peut de même mettre en oeuvre des silicones non volatiles comme les huiles et gommes polydiméthylsiloxanes et α,ω-bis(hydroxy) polydiméthylsiloxanes ainsi que les gommes polydiméthylsiloxanes, polyphénylméthylsiloxane et α,ω-bis(hydroxy)poly diméthylsiloxanes peuvent de même être utilisés.

On préfère plus particulièrement les huiles α,ω-bis(triméthyl)polydiméthyl siloxanes, les huiles α,ω-bis(hydroxy)polydiméthyl siloxanes.

A titre représentatif de silicones convenant tout particulièrement à la présente invention, on peut notamment citer les silicones de type polydiméthylsiloxane (diméthicone) et diphényldiméthicone.

Un autre type de silicones aminés convenant particulièrement bien à la présente invention, sont les silicones à groupes pipéridinyles stériquement encombrés.
Les groupes pipéridinyles stériquement encombrés peuvent être représentés par les formules I est II ci-après :
- les groupes pipéridinyles stériquement encombrés de formule I 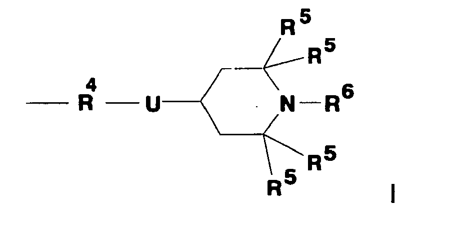 où
   * R⁴ est un radical hydrocarboné divalent choisi parmi :
      ◆ les radicaux alkylènes linéaires ou ramifiés, ayant 2 à 18 atomes de carbone;
      ◆ les radicaux alkylène-carbonyle dont la partie alkylène linéaire ou ramifiée, comporte 2 à 20 atomes de carbone;
      ◆ les radicaux alkylène-cyclohexylène dont la partie alkylène linéaire ou ramifiée, comporte 2 à 12 atomes de carbone et la partie cyclo-hexylène comporte un groupement OH et éventuellement 1 ou 2 radicaux alkyles ayant 1 à 4 atomes de carbone;
      ◆ les radicaux de formule -R⁷ - O - R⁷ dans laquelle les radicaux R⁷ identiques ou différents représentent des radicaux alkylènes ayant 1 à 12 atomes de carbone;
      ◆ les radicaux de formule -R⁷ - O - R⁷ dans laquelle les radicaux R⁷ ont les significations indiquées précédemment et l'un d'entre eux ou les deux sont substitués par un ou deux groupement(s) -OH;
      ◆ les radicaux de formule -R⁷ - COO - R⁷ dans laquelle les radicaux R⁷ ont les significations indiquées précédemment;
      ◆ les radicaux de formule -R⁸ -O -R⁹ - O-CO-R⁸ dans laquelle les radicaux R⁸ et R⁹ identiques ou différents, représentent des radicaux alkylènes ayant 2 à 12 atomes de carbone et le radical R⁹ est éventuellement substitué par un radical hydroxyle;
      ◆ U représente -O- ou -NR¹⁰-, R¹⁰ étant un radical choisi parmi un atome d'hydrogène, un radical alkyle linéaire ou ramifié comportant 1 à 6 atomes de carbone et un radical divalent de formule : 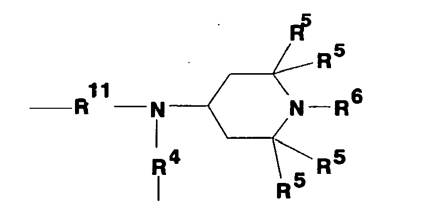
      dans laquelle R⁴ a la signification indiquée précédemment, R⁵ et R⁶ ont les significations indiquées ci-après et R¹¹ représente un radical divalent alkylène, linéaire ou ramifié, ayant de 1 à 12 atomes de carbone, l'un des liens valentiels (celui de R¹¹) étant relié à l'atome de -NR¹⁰-, l'autre (celui de R⁴) étant relié à un atome de silicium;
   * les radicaux R⁵ sont identiques ou différents, choisis parmi les radicaux alkyles linéaires ou ramifiés ayant 1 à 3 atomes de carbone et le radical phényle;
   * le radical R⁶ représente un radical hydrogène ou le radical R⁵ ou O•.
- ou les groupes pipéridinyles stériquement encombrés de formule II 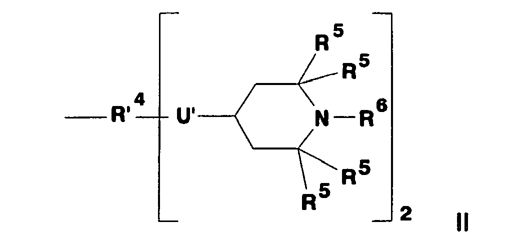 où
   ◆ R'⁴ est choisi parmi un radical trivalent de formule : 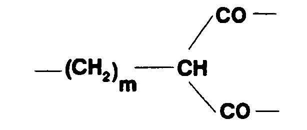 où m représente un nombre de 2 à 20,
      et un radical trivalent de formule : 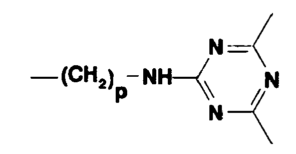 où p représente un nombre de 2 à 20;
   ◆ U' représente -O- ou NR¹²-, R¹² étant un radical choisi parmi un atome d'hydrogène, un radical alkyle linéaire ou ramifié comportant 1 à 6 atomes de carbone;
   *R⁵ et R⁶ ont les mêmes significations que celles données ci-dessus à propos de la formule I.

D'une manière préférentielle, lesdits polyorganosiloxanes à fonction pipérydinyle stériquement encombrée sont notamment ceux pouvant être préparés selon le procédé décrit dans EP-A-659930.
Tout préférentiellement, ledit polyorganosiloxane à fonction pipérydinyle stériquement encombrée est un polyorganosiloxane linéaire, cyclique ou tridimensionnel de formule : 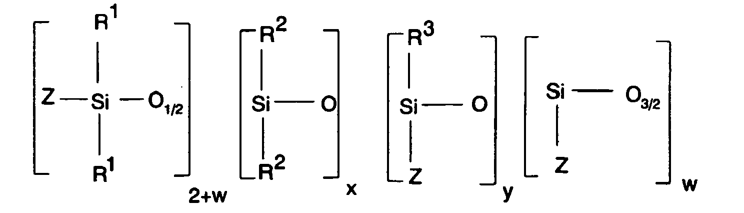 dans laquelle :
**(1)** les symboles Z, identiques ou différents, représentent R¹ ci-dessous et/ou B;
**(2)** les symboles R¹, R² et R³, identiques et/ou différents, représentent un radical hydrocarboné monovalent choisi parmi les radicaux alkyles linéaires ou ramifiés ayant de 1 à 4 atomes de carbone, les radicaux alkoxy linéaires ou ramifiés ayant de 1 à 4 atomes de carbone, un radical phényle et, de préférence un radical hydroxy, un radical éthoxy, un radical méthoxy ou un radical méthyle;
**(3)** les symboles **B**, groupements fonctionnels identiques et/ou différents, représentent un groupement à fonction(s) pipéridinyle(s) stériquement encombrée(s) choisi parmi ceux mentionnés ci-dessus ; et
**(4)** - le nombre de motifs ηSi sans groupement B va de 10 à 450, de préférence de 50 à 250 ;
   - le nombre de motifs ηSi avec un groupement B va de 1 à 5, de préférence de 1 à 3 ;
   - 0 ≤ w ≤ 10 et 8 < y < 448.

De manière toute préférentielle, ledit polyorganosiloxane à fonction(s) pipéridinyle(s) stériquement encombrée(s) est linéaire.

Enfin, la phase organique peut comprendre une quantité d'eau qui ne dépasse pas la limite de solubilité de l'eau dans ladite phase organique (à une température comprise entre 20°C et la température de préparation de l'émulsion).

La phase organique peut en outre comprendre au moins une matière active.

Lesdites matières actives se présentent sous forme liquide, soluble dans la phase organique, solubilisée dans un solvant organique miscible dans la phase organique, ou encore sous la forme d'un solide en dispersion dans ladite phase.

Plus particulièrement, les matières actives sont telles que leur solubilité dans l'eau ne dépasse pas 10 % en poids entre 20°C et la température de préparation de l'émulsion.

En outre, les matières actives présentent de préférence une température de fusion inférieure ou égale à 100°C, plus particulièrement inférieure ou égale à 80°C.

A titre d'exemple de matières actives dans le domaine de la cosmétique on peut citer les huiles silicones appartenant par exemple à la famille des diméthicones ; les vitamines lipophiles, comme la vitamine A.

Dans le domaine du papier, on peut citer à titre d'exemples les résines de collage et d'hydrofugation, telles que le dimère d'alkylcétène (AKD) ou l'anhydride alcényle succinique (ASA).

Dans le domaine de la détergence, on peut mentionner en tant que matières actives possibles les antimousses silicones, les huiles minérales et végétales, et les silicones aminés.

Dans le domaine de l'agrochimie, les matières actives phytosanitaires peuvent être choisies parmi la famille des α-cyano-phénoxybenzyl carboxylates ou des α-cyano-halogénophénoxy-carboxylates, la famille des N-méthylcarbonates comprenant des substituants aromatiques, les matières actives telles que Aldrin, Azinphos-methyl, Benfluralin, Bifenthrin, Chlorphoxim, Chlorpyrifos, Fluchloralin, Fluroxypyr, Dichlorvos, Malathion, Molinate, Parathion, Permethrin, Profenofos, Propiconazole, Prothiofos, Pyrifenox, Butachlor, Metolachlor, Chlorimephos, Diazinon, Fluazifop-P-butyl, Heptopargil, Mecarbam, Propargite, Prosulfocarb, Bromophos-ethyl, Carbophenothion, Cyhalothrin.

Il est de même possible d'utiliser des matières actives telles que celles entrant dans la composition de lubrifiants pour le travail ou la déformation des matériaux. La matière active est habituellement une huile, un dérivé d'une huile ou encore un ester d'acide gras.

La matière active peut aussi être choisie parmi les solvants organiques ou les mélanges de tels solvants pas ou peu miscibles dans l'eau, comme notamment ceux mis en oeuvre pour le nettoyage ou le décapage, tels que les coupes pétrolières aromatiques, les composés terpéniques comme les D- ou L- limonènes, ainsi que les solvants comme le Solvesso^{®}. Conviennent aussi comme solvants, les huiles hydrocarbonées comme l'huile de vaseline, et les solvants chlorés, les diesters d'alkyle en C₁-C₄ d'au moins un diacide aliphatique en C₄-C₆. On met en oeuvre plus particulièrement des mélanges d'esters de diacides qui sont des esters dérivés essentiellement des acides adipique, glutarique et succinique, les groupes alkyles de la partie ester étant surtout choisis parmi les groupes méthyle et éthyle, mais pouvant être également propyle, isopropyle, butyle, n-butyle et isobutyle ; l'anisole ; la n-méthylpyrrolidone ; le diméthylsulfoxyde ; les cétones comme la cyclopentanone, la méthylisobutylcétone ; les polyalkylène glycols, comme le polyéthylène glycol 400, le polypropylène glycol 400.

Au cas où la phase organique comprend une ou plusieurs matières actives hydrophobes différentes de la phase organique, leur teneur représente plus particulièrement 10 à 50 % en poids de ladite phase organique interne.

Enfin, la phase organique elle-même, telle qu'elle a été décrite auparavant, peut être considérée comme matière active hydrophobe.

En outre, la phase aqueuse externe peut comprendre au moins un tensioactif et/ou au moins un polymère amphiphile externes.

Plus particulièrement, les tensioactifs et polymères satisfont la règle de Bancroft énoncée plus haut.

Selon une première variante, le tensioactif et/ou polymère amphiphiles externes sont choisis parmi les tensioactifs non ioniques, les polymères amphiphiles non ioniques, éventuellement associé(s) à au moins un tensioactif anionique et/ou au moins un polymère amphiphile anionique.

Selon cette première variante, la teneur totale en tensioactif(s) et/ou polymère(s) amphiphile(s) externe est comprise entre 0,5 et 10 % en poids, de préférence entre 1 et 5 % en poids, par rapport à l'émulsion inverse ; la quantité en tensioactif et/ou polymère amphiphile anioniques, s'ils sont présents, représente 0,5 à 5 % en poids, de préférence 0,5 à 2 % en poids, par rapport au poids de tensioactif et/ou polymère amphiphile non ioniques.

En ce qui concerne les tensioactifs non ioniques polyalcoxylés, on utilise des composés pour lesquels le nombre de motifs alcoxylés, plus particulièrement éthoxylés et/ou propoxylés, est tel que la valeur de HLB soit supérieure ou égale à 10.

A titre illustratif, conviennent, seuls ou en mélange, les tensioactifs suivants, :
- les alcools gras alcoxylés
- les triglycérides alcoxylés
- les acides gras alcoxylés
- les esters de sorbitan alcoxylés
- les amines grasses alcoxylées
- les di(phényl-1 éthyl) phénols alcoxylés
- les tri(phényl-1 éthyl) phénols alcoxylés
- les alkyls phénols alcoxylés.

Les alcools gras alcoxylés comprennent généralement de 6 à 22 atomes de carbone, les motifs alcoxylés étant exclus de ces nombres.

Les triglycérides alcoxylés peuvent être des triglycérides d'origine végétale ou animale.

Les esters de sorbitan alcoxylés sont des esters du sorbitol cyclisés d'acide gras comprenant de 10 à 20 atomes de carbone comme l'acide laurique, l'acide stéarique ou l'acide oléïque.

Les amines grasses alcoxylées ont généralement de 10 à 22 atomes de carbone, les motifs alcoxylés étant exclus de ces nombres.

Les alkylphénols alcoxylés ont généralement un ou deux groupes alkyles, linéaires ou ramifiés, ayant 4 à 12 atomes de carbone. A titre d'exemple on peut citer notamment les groupes octyle, nonyle ou dodécyle.

En ce qui concerne les polymères amphiphiles non ioniques, on utilise de préférence des composés polyalcoxylés, comprenant au moins deux blocs, l'un d'eux étant hydrophile, l'autre hydrophobe ; l'un au moins des blocs comprenant des motifs polyalcoxylés, plus particulièrement polyéthoxylés et/ou polypropoxylés.

Ce qui a été indiqué auparavant dans le cadre de la description des monomères hydrophiles non ioniques et des monomères hydrophobes utilisables pour la préparation des copolymères à blocs entrant dans la composition de l'émulsion, reste valable et ne sera pas repris ici.

A titre d'exemples de polymères de ce type, on peut citer entre autres les polymères triblocs polyéthylène glycol / polypropylène glycol / polyéthylène glycol. De tels polymères sont bien connus et sont notamment commercialisés sous les marques Pluronic (commercialisé par BASF), Arlatone (commercialisé par ICI).

Selon un autre mode de réalisation, le polymère amphiphile non ionique est un polymère amphiphile à blocs, obtenu par polymérisation d'au moins un monomère hydrophile non ionique et d'au moins un monomère hydrophobe, la proportion et la nature desdits monomères étant telles que le polymère résultant vérifie les conditions de la règle de Bancroft.

Ces polymères amphiphiles comprennent de plus au moins un bloc hydrophobe et au moins un bloc hydrophile non ionique.

Au cas où ledit polymère comprend au moins trois blocs, et plus particulièrement trois blocs, le polymère est avantageusement linéaire. En outre, les blocs hydrophiles se trouvent plus particulièrement aux extrémités.

Au cas où les polymères comprennent plus de trois blocs, ces derniers sont de préférence sous la forme de polymères greffés ou peignes.

Les listes des monomères hydrophiles non ioniques, des monomères hydrophobes non ioniques, de même que les diverses méthodes de préparation, citées dans le cadre de la description des polymères amphiphiles à blocs, peuvent être reprise dans le cas des polymères selon cette variante.

Toutefois les monomères hydrophiles préférés sont l'acrylamide et le méthacrylamide, seuls ou en mélange, ou sous la forme de macromonomères ; les monomères préférés sont les esters de l'acide acryliques avec les alcools linéaires ou ramifiés en C₁-C₄ tels que l'acrylate de méthyle, d'éthyle, de propyle et de butyle, les esters vinyliques comme l'acétate de vinyle, le styrène, l'α-méthylstyrène.

Le tensioactif et/ou polymère amphiphile non ioniques qui viennent d'être décrits peuvent être associés à au moins un tensioactif et/ou polymère amphiphile anioniques, parmi lesquels peuvent être utilisés, seuls ou en mélanges :
- les alkylesters sulfonates, par exemple de formule R-CH(SO₃M)-COOR', où R représente un radical alkyle en C₈-C₂₀, de préférence en C₁₀-C₁₆, R' un radical alkyle en C₁-C₆, de préférence en C₁-C₃ et M un cation alcalin (sodium, potassium, lithium), ammonium substitué ou non substitué (méthyl-, diméthyl-, triméthyl-, tétraméthylammonium, diméthylpipéridinium ...) ou dérivé d'une alcanolamine (monoéthanolamine, diéthanolamine, triéthanolamine ...). On peut citer tout particulièrement les méthyl ester sulfonates dont le radical R est en C₁₄-C₁₆ ; les alkylbenzènesulfonates, plus particulièrement en C₉-C₂₀, les alkylsulfonates primaires ou secondaires, notamment en C₈-C₂₂, les alkylglycérol sulfonates ;
- les alkylsulfates par exemple de formule ROSO₃M, où R représente un radical alkyle ou hydroxyalkyle en C₁₀-C₂₄, de préférence en C₁₂-C₂₀ ; M représentant un atome d'hydrogène ou un cation de même définition que ci-dessus, ainsi que leurs dérivés polyalcoxylés (éthoxylés et/ou propoxylés), comme par exemple le dodécylsulfate de sodium ;
- les alkyléthersulfates par exemple de formule RO(CH₂CH_{2O})ₙSO₃M où R représente un radical alkyle ou hydroxyalkyle en C₁₀-C₂₄, de préférence en C₁₂-C₂₀ ; M représentant un atome d'hydrogène ou un cation de même définition que ci-dessus, n variant généralement de 1 à 4, ainsi que leurs dérivés polyalcoxylés (éthoxylés et/ou propoxylés), comme par exemple le laurylethersulfate avec n = 2.
- les alkylamides sulfates, par exemple de formule RCONHR'OSO₃M où R représente un radical alkyle en C₂-C₂₂, de préférence en C₆-C₂₀, R' un radical alkyle en C₂-C₃, M représentant un atome d'hydrogène ou un cation de même définition que ci-dessus, ainsi que leurs dérivés polyalcoxylés (éthoxylés et/ou propoxylés) ;
- les sels d'acides gras saturés ou insaturés, par exemple comme ceux en C₈-C₂₄, de préférence en C₁₄-C₂₀, les N-acyl N-alkyltaurates, les alkyliséthionates, les alkylsuccinamates et alkylsulfosuccinates, les monoesters ou diesters de sulfosuccinates, les N-acyl sarcosinates, les polyéthoxycarboxylates ; et
- les phosphates esters d'alkyle et/ou d'alkyléther et/ou d'alkylaryléther.

Il est précisé qu'au cas où une espèce anionique (tensioactif) est mise en contact du copolymère à blocs, que ce soit lors de la préparation de l'émulsion, après que celle-ci soit fabriquée ou encore lors de la préparation de la formulation dans laquelle l'émulsion est introduite, le copolymère à blocs et l'espèce anionique sont de nature telle et utilisés en concentration telle qu'il sont compatibles. Le terme compatible signifie qu'il n'y a pas de séparation macroscopique de phase à 20°C au bout d'une heure.

Parmi les polymères anioniques susceptibles d'être employés, on peut citer tout particulièrement les polymères à blocs, de préférence diblocs ou triblocs, obtenus par polymérisation d'au moins un monomère hydrophile anionique, éventuellement d'au moins un monomère hydrophile non ionique, et d'au moins un monomère hydrophobe.

Là encore, le choix, des monomères et leurs proportions respectives sont telles que le polymère résultant vérifie les conditions de la règle de Bancroft.

Les monomères hydrophiles non ioniques, anioniques, les monomères hydrophobes ainsi que les modes de synthèse cités dans le cadre de la description des polymères amphiphiles internes peuvent être mis en oeuvre pour l'obtention des polymères amphiphiles externes selon cette variante. On pourra donc s'y référer.

Une deuxième variante de l'invention consiste à mettre en oeuvre en tant que tensioactif et/ou polymère amphiphile externe, un ou plusieurs polymères amphiphiles anioniques éventuellement associés à au moins un tensioactif anionique ; la teneur totale en polymère amphiphile et/ou tensioactif anioniques est comprise entre 0,5 et 10 % en poids, de préférence entre 1 et 5 % en poids, par rapport à l'émulsion inverse.

On pourra se reporter aux listes indiquées pour la première variante de tensioactifs et/ou polymères externes pour ce qui concerne les tensioactifs et/ou polymères conformes à cette variante.

Par ailleurs, le rapport pondéral phase organique / phase aqueuse est plus particulièrement compris entre 0,1/99,9 et 90/10.

Conformément à un deuxième mode de réalisation de l'invention, le copolymère à blocs qui vient d'être décrit est utilisé comme aide au dépôt d'une émulsion multiple.

Selon ce mode de réalisation de l'invention, une première variante a pour objet des émulsions multiples comprenant une phase aqueuse interne dispersée dans la phase organique, l'émulsion inverse (ensemble de la phase aqueuse interne et de la phase organique), étant dispersée dans la phase aqueuse. De plus, la phase aqueuse interne et la phase organique comprennent au moins un tensioactif et/ou au moins un polymère amphiphile internes.

La phase aqueuse interne peut comprendre éventuellement au moins une matière active hydrophile.

Cette matière active hydrophile peut se présenter sous une forme liquide ; sous une forme solubilisée dans un solvant miscible à l'eau comme l'éthanol, le propylène glycol, le glycérol ; ou encore sous une forme solide.

La teneur en matière active hydrophile est plus particulièrement comprise entre 0,1 et 50 % en poids de la phase aqueuse interne, et de préférence comprise entre 0,1 et 20 % en poids de la phase aqueuse interne.

A titre d'exemple de matières actives utilisables dans le domaine de la cosmétique, on peut citer les substances qui ont un effet cosmétique, un effet thérapeutique ou toute autre substance utilisable pour le traitement de la peau et du cheveu.

Ainsi, on peut utiliser, en tant que matière active des agents conditionneurs de la peau et du cheveu, comme notamment les polymères comprenant des ammonium quaternaires qui peuvent éventuellement être engagés dans des hétérocycles (composés du type des quaternium, polyquaternium, etc.)., des agents humectants ; des agents fixants (styling) qui sont plus particulièrement choisis parmi des polymères (homo-, co- ou ter-polymères par exemple acrylamide, acrylamide/acrylate de sodium, polystyrène sulfonate, etc.), les polymères cationiques, la polyvinylpyrrolidone, l'acétate de polyvinyle,etc.

Il est de même possible d'utiliser des agents colorants ; des agents astringents, utilisables dans les déodorants et qui sont plus particulièrement des sels d'aluminium, de zirconium ; des agents antibactériens ; des agents anti-inflammatoires, des agents anesthésiants, des filtres solaires, etc.

On peut aussi citer les α- et β- hydroxyacides, comme les acides citrique, lactique, glycolique, salicylique ; les acides dicarboxyliques de préférence insaturés et comprenant 9 à 16 atomes de carbone comme l'acide azélaique ; la vitamine C et ses dérivés, notamment les dérivés glycosylés et phosphatés ; les biocides notamment cationiques (Glokill PQ, Rhodoquat RP50, commercialisés par Rhodia Chimie), comme matières actives convenables dans les formulations cosmétiques.

A titre de matières actives convenables dans le domaine de la fabrication du papier, on peut citer notamment le chlorure de calcium, l'acide chlorhydrique.

Conformément à un mode de réalisation particulièrement avantageux, la phase aqueuse interne peut comprendre au moins un additif choisi parmi les sels tels que les halogénures de métaux alcalins ou alcalino-terreux (comme le chlorure de sodium, le chlorure de calcium), ou les sulfates de métaux alcalin ou alcalino-terreux (comme le sulfate de sodium), ou leurs mélanges. A titre d'additif possible de la phase aqueuse interne, on peut aussi citer les sucres, comme le glucose par exemple, ou encore les polysaccharides, comme notamment le dextran, ou leurs mélanges.

La concentration en additif du type sel, lorsque ce dernier est présent, est plus particulièrement comprise entre 0,05 et 1 mol/l, de préférence 0,1 à 0,4 mol/l.

La concentration en additif du type sucre et/ou polysaccharide est telle que la pression osmotique de la phase aqueuse interne comprenant le sucre et/ou polysaccharide correspond à la pression osmotique d'une phase aqueuse interne comprenant 0,05 à 1 mol/l de sel.

Pour ce qui concerne la phase organique, et plus particulièrement sa nature ainsi que celle de la ou des matières actives qu'elle peut comprendre, on pourra se reporter à ce qui a été décrit à ce sujet dans la partie de la description relative aux émulsions simples.

Comme indiqué auparavant, la phase organique et la phase aqueuse interne comprennent au moins un tensioactif et/ou polymère amphiphile internes.

Selon une première possibilité, ledit tensioactif et/ou polymère satisfont à la règle de Bancroft. En d'autres termes, la fraction du tensioactif ou du polymère soluble dans la phase continue de l'émulsion inverse est supérieure à la fraction soluble dans la dispersée de l'émulsion inverse.

Plus particulièrement, le tensioactif et/ou polymère amphiphiles sont choisis parmi les tensioactifs non ioniques et/ou les polymères amphiphiles à blocs,

A titre d'exemples de tensioactifs non ionique susceptibles d'entrer dans la composition de l'émulsion inverse, on peut citer, seuls ou en mélange, les tensioactifs choisis parmi :
- les alcools gras alcoxylés
- les mono-, di- et triglycérides alcoxylés
- les acides gras alcoxylés
- les esters de sorbitan éventuellement alcoxylés
- les amines grasses alcoxylées
- les di(phényl-1 éthyl) phénols alcoxylés
- les tri(phényl-1 éthyl) phénols alcoxylés
- les alkyls phénols alcoxylés
le nombre de motifs alcoxylés (éthoxylés, propoxylés, butoxylés) est tel que la valeur de HLB soit inférieure ou égale à 8.

Quant au polymère amphiphile à blocs, celui-ci comprend au moins deux blocs, dont l'un au moins est un bloc hydrophobe et l'un au moins est un bloc hydrophile neutre, anionique ou cationique.

Au cas où le polymère amphiphile comprend au moins trois blocs, et plus particulièrement trois blocs, le polymère est de préférence linéaire. En outre, les blocs hydrophobes se trouvent plus particulièrement aux extrémités.

Au cas où les polymères comprennent plus de trois blocs, ces derniers sont de préférence sous la forme de polymères greffés ou peignes.

Ces polymères amphiphiles internes peuvent être synthétisés en mettant en oeuvre les méthodes de polymérisation mentionnées dans le cadre de la description du copolymère à blocs., et l'on pourra de plus se reporter aux diverses listes de monomères hydrophobes non ioniques, hydrophiles non ioniques, cationiques détaillées à cette occasion.

A titre de monomère hydrophobes non ioniques, on peut notamment citer :
- les esters des acides mono- ou poly- carboxyliques, linéaires, ramifiés, cycliques ou aromatiques, comprenant au moins une insaturation éthylénique,
- les esters d'acides carboxyliques saturés comprenant 8 à 30 atomes de carbone, éventuellement porteurs d'un groupement hydroxyle ;
- les nitriles αβ-éthyléniquement insaturés, les éthers vinyliques, les esters vinyliques, les monomères vinylaromatiques, les halogénures de vinyle ou de vinylidène,
- les monomères hydrocarbonés, linéaires ou ramifiés, aromatiques ou non, comprenant au moins une insaturation éthylénique,
- les monomères de type siloxane cyclique ou non, les chlorosilanes ;
- l'oxyde de propylène, l'oxyde de butylène ;
seuls ou en mélanges, ainsi que les macromonomères dérivant de tels monomères.

Pour ce qui a trait aux monomères hydrophiles non ioniques, convient notamment l'oxyde d'éthylène ; les amides des acides mono- ou poly- carboxyliques, linéaires, ramifiés, cycliques ou aromatiques, comprenant au moins une insaturation éthylénique ou dérivés, comme le (méth)acrylamide, le N-méthylol (méth)acrylamide ; les esters hydrophiles dérivant de l'acide (méth)acrylique comme par exemple le (méth)acrylate de 2-hydroxyéthyle ; les esters vinyliques permettant d'obtenir des blocs alcool polyvinylique après hydrolyse, comme l'acétate de vinyle, le Versatate^{®} de vinyle, le propionate de vinyle ; les monomères du type des sucres comme les osides, les polyholosides fortement dépolymérisés.

Quant aux monomères, ils peuvent être avantageusement choisis parmi :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, aromatique ou non, la vinylamine, l'éthylène imine ;
- les sels d'ammonium de diallyldialkyl ;
seuls ou en mélanges, ou les sels correspondants, ainsi que les macromonomères dérivant de tels monomères.

Ces monomères ont été décrits auparavant, et l'on pourra se reporter aux listes.

En ce qui concerne les monomères hydrophiles anioniques à partir desquels ces polymères amphiphiles internes peuvent être obtenus, on peut mentionner, par exemple les monomères comprenant au moins une fonction carboxylique, sulfonique, sulfurique, phosphonique, phosphorique, sulfosuccinique, ou les sels correspondants.

Plus particulièrement, les monomères sont choisis parmi :
- les acides mono- ou poly- carboxyliques linéaires, ramifiés, cycliques ou aromatiques, les dérivés N-substitués de tels acides ; les monoesters d'acides polycarboxyliques, comprenant au moins une insaturation éthylénique ;
- les acides vinyl carboxyliques linéaires, ramifiés, cycliques ou aromatiques ;
- les aminoacides comprenant une ou plusieurs insaturations éthyléniques ;
seuls ou en mélanges, leurs précurseurs, leurs dérivés sulfoniques ou phosphoniques, ainsi que les macromonomères dérivant de tels monomères ; les monomères ou macromonomères pouvant être sous la forme de sels.

A titre d'exemples de monomères anioniques, on peut citer sans intention de s'y limiter :
- l'acide acrylique, l'acide méthacrylique, l'acide fumarique, l'acide itaconique, l'acide citraconique, l'acide maléique, l'acide acrylamido glycolique, l'acide 2-propène 1-sulfonique, l'acide méthallyl sulfonique, l'acide styrène sulfonique, l'acide α-acrylamido méthylpropane sulfonique, le 2-sulfoéthylène méthacylate, l'acide sulfopropyl acrylique, l'acide bis-sulfopropyl acrylique, l'acide bis-sulfopropyl méthacrylique, l'acide sulfatoéthyl méthacrylique, le monoester phosphate d'acide hydroxyéthyl méthacrylique, ainsi que les sels de métal alcalin, comme le sodium, le potassium, ou d'ammonium ;
- l'acide vinyl sulfonique, l'acide vinylbenzène sulfonique, l'acide vinyl phosphonique, l'acide vinylidène phosphorique, l'acide vinyl benzoïque, ainsi que les sels de métal alcalin, comme le sodium, le potassium, ou d'ammonium ;
- le N-méthacryloyl alanine, le N-acryloyl-hydroxy-glycine ;
seuls ou en mélanges, ainsi que les macromonomères dérivant de tels monomères.

On ne sortirait pas du cadre de la présente invention en mettant en oeuvre des monomères précurseurs de ceux qui viennent d'être cités. En d'autres termes, ces monomères présentent des motifs qui, une fois incorporés dans la chaîne polymère, peuvent être transformés, notamment par un traitement chimique tel que l'hydrolyse, pour redonner les espèces anioniques précitées. Par exemple, les monomères totalement ou partiellement estérifiés des monomères précités peuvent être mis en oeuvre pour être, par la suite, hydrolysés totalement ou en partie.

De préférence, les polymères amphiphiles internes présentent une masse molaire en poids inférieure ou égale à 100000 g/mol, plus particulièrement comprise entre 1000 et 50000 g/mol, de préférence entre 1000 et 20000 g/mol. Il est précisé que les masses molaires en poids indiquées ci-dessus sont des masses molaires théoriques, évaluées en fonction des quantités respectives des monomères introduites lors de la préparation desdits polymères.

De préférence, on met en oeuvre un polymère amphiphile à bloc de type non ionique.

A titre d'exemple de polymère amphiphile à blocs convenant à la mise en oeuvre de l'invention, on peut citer les polymères triblocs polyhydroxystéarate - polyéthylène glycol - polyhydroxystéarate (les produits de la gamme Arlacel de ICI en sont un exemple), les polymères à blocs polydiméthylsiloxane greffé polyéther polyalkyle (comme les produits de la marque Tegopren commercialisé par Goldschmidt).

Selon une deuxième possibilité, on met en oeuvre, comme tensioactif interne, au moins un tensioactif cationique. Dans le cas de cette deuxième variante, le tensioactif interne ne satisfait pas la règle de Bancroft précédemment énoncée. En effet, le tensioactif cationique est soluble dans la phase dispersée et non dans la phase continue de l'émulsion inverse.

Parmi les tensioactifs cationiques convenables, on peut notamment utiliser les amines grasses aliphatiques ou aromatiques, les amides gras aliphatiques, les dérivés d'ammonium quaternaire (Rhodoquat RP50 de Rhodia Chimie).

Enfin, une troisième variante de l'invention consiste à combiner les deux possibilités qui viennent d'être détaillées.

Quelle que soit la variante retenue, la teneur totale en tensioactif et/ou polymère amphiphile internes représente plus particulièrement de 0,1 à 10 % en poids, de préférence de 2 à 10 % en poids par rapport à la phase aqueuse interne.

En outre, l'émulsion inverse présente plus particulièrement un rapport pondéral phase aqueuse interne / phase organique compris entre 10/90 et 90/10. De préférence, un rapport pondéral phase aqueuse / phase organique est compris entre 30/70 et 80/20.

Ainsi que cela a déjà été mentionné, l'ensemble phase aqueuse interne et phase organique est dispersé dans une phase aqueuse.

Ladite phase aqueuse peut éventuellement comprendre au moins une matière active.

On pourra se référer à ce qui a été indiqué auparavant concernant la nature et la quantité de matière active soluble en phase aqueuse.

En outre, l'émulsion qui vient d'être décrite peut éventuellement comprendre au moins un tensioactif et/ou au moins un polymère amphiphile externe.

Là encore, ce qui a été détaillé concernant la nature du tensioactif / polymère amphiphile, les diverses variantes et les teneurs respectives, dans le cadre de la phase aqueuse d'une émulsion simple, est valable dans le cas de cette variante, et l'on pourra s'y reporter.

En outre, la phase aqueuse externe peut comprendre au moins un additif dont l'un des rôles est d'équilibrer les pressions osmotiques de la phase aqueuse externe et de la phase aqueuse interne de l'émulsion multiple. Parmi les additifs envisageables, on peut citer les sels choisis parmi les halogénures de métaux alcalins ou alcalino-terreux (comme le chlorure de sodium, le chlorure de calcium), au moins un sulfate de métal alcalin ou alcalino-terreux (comme le sulfate de sodium) ou leurs mélanges ; les sucres (glucose par exemple), ou encore les polysaccharides (notamment le dextran) ou leurs mélanges. La phase aqueuse externe peut comprendre une combinaisons de tous ces additifs.

Les concentrations en sel, en sucre et/ou en polysaccharide sont telles que les pressions osmotiques des phases aqueuses externe et interne sont équilibrées.

Selon une variante avantageuse de la présente invention, la phase aqueuse de l'émulsion peut comprendre au moins un polymère épaississant. Ce polymère a pour effet d'éviter le crémage et/ou la sédimentation de l'émulsion finale.

A titre d'illustration, on peut utiliser des polymères épaississants extraits de végétaux et éventuellement modifiés, tels que les carraghénannes, les alginates, les carboxyméthyl celluloses, les méthylcelluloses, les hydroxypropyl celluloses, les hydroxyéthyl celluloses, les gellanes.

On peut de même employer des polymères épaississants du type des polysaccharides d'origine animale, végétale, bactérienne, on peut citer à titre d'exemple non limitatif, la gomme xanthane, le guar et dérivés (tels que l'hydroxypropyl guar par exemple), les polydextroses, ou leurs combinaisons.

Lorsqu'il est présent, la teneur en polymère épaississant est plus particulièrement comprise entre 0,1 et 2 % en poids par rapport à la phase aqueuse, de préférence entre 0,1 et 0,5 % en poids par rapport à la phase aqueuse.

Le rapport pondéral de l'ensemble (phase aqueuse interne et phase organique) / phase aqueuse représente 0.1%/99.9% à 90/10.

Selon ce mode de réalisation de l'invention dans lequel l'émulsion se trouve sous la forme d'une émulsion multiple, une deuxième variante a pour objet des émulsions multiples comprenant une phase organique interne dispersée dans la phase organique, l'ensemble de la phase organique interne et de la phase organique étant dispersé dans la phase aqueuse. De plus, la phase organique interne et la phase organique comprennent au moins un tensioactif et/ou au moins un polymère amphiphile interne.

Ce qui a été indiqué précédemment dans le texte, concernant la nature des phases organiques reste valable et en sera pas repris ici.

Il est simplement précisé que la phase organique et la phase organique interne sont choisies de telle façon qu'elles ne soient pas ou peu miscibles entre elles. Ainsi, la nature de ces deux phases est choisie de telle sorte que la solubilité de l'une dans l'autre ne dépasse pas 10 % en poids à une température comprise entre 20°C et la température de préparation de l'émulsion.

De préférence selon ce mode de réalisation, l'une des deux phases organiques est une phase silicone. De préférence, la phase silicone est la phase organique.

Par ailleurs, optionnellement, au moins l'une des phases organique et organique interne peut comprendre au moins une matière active.

La description relative à la nature et à la teneur de matière actives de ce type, entrant dans la composition de la phase organique d'une émulsion simple peut être reprise ici.

L'émulsion phase organique interne/ phase organique comprend au moins un polymère stabilisant interne.

Plus précisément, ledit polymère stabilisant remplit les conditions de la règle de Bancroft définie auparavant.

Il est précisé à titre simplement indicatif, que la masse molaire en nombre du copolymère est inférieure ou égale à 100000 g/mol, plus particulièrement comprise entre 1000 et 50000 g/mol. La masse molaire en nombre est ici donnée avec une valeur absolue, et peut être avantageusement déterminée par chromatographie par exclusion stérique couplée à la méthode MALLS.

De manière avantageuse, si chacun des blocs du copolymère représentait un polymère (même taille et composition que les blocs), alors les monomères constituant chacun des blocs seraient choisis de telle sorte que chaque polymère soit soluble, dans les conditions de température et de concentrations mentionnées ci-dessus, soit dans la phase organique interne (pour le polymère dérivant des blocs solubles dans cette phase) soit dans la phase organique (pour le polymère dérivant des blocs solubles dans cette phase).

Plus particulièrement, la fraction du copolymère qui est soluble dans la phase silicone dérive d'un polysiloxane pouvant être choisi parmi les silicones listées dans le cadre des composés utilisables en tant que phase organique silicone.

De préférence, le polysiloxane est porteur de fonctions réactives, comme les fonctions -OH, -NH₂, entre autres.

En ce qui concerne la fraction soluble dans la phase organique non silicone, celle-ci dérive, de préférence, de la polymérisation d'au moins un monomère choisi parmi les monomères suivants :
- les esters des acides mono- ou poly- carboxyliques, linéaires, ramifiés, cycliques ou aromatiques, comprenant au moins une insaturation éthylénique ;
- les nitriles αβ-éthyléniquement insaturés, les éthers vinyliques, les esters vinyliques, les monomères vinylaromatiques, les halogénures de vinyle ou de vinylidène ;
- les monomères hydrocarbonés, linéaires ou ramifiés, aromatiques ou non, comprenant au moins une insaturation éthylénique ;
seuls ou en mélanges, ainsi que les macromonomères dérivant de tels monomères.

Il est à noter que la fraction du copolymère qui est soluble dans cette phase non silicone peut être obtenue à partir des monomères précités, combinés à des monomères de nature chimique différente, comme par exemple des monomères hydrophiles, non ionique ou ioniques.

Selon un mode de réalisation avantageux, le copolymère est un copolymère linéaire à blocs, de préférence, comprenant au moins trois blocs.

Les copolymères utilisables peuvent être obtenus, de manière avantageuse, par voie radicalaire, de préférence contrôlée.

De préférence, on peut obtenir de tels polymères en mettant en oeuvre un procédé de préparation par activation thermique de copolymères hybrides silicones et organiques comprenant des motifs (I) :

RₓU_{y}SiO_{[4-(x+y)]/2} (I)

dans lesquels:
- x est égal à 0, 1, 2 ou 3, y est égal à 0, 1, 2 ou 3 avec 2 s (x+y) ≤ 3 et y est différent de 0 pour au moins un des motifs du copolymère hybride,
   • les symboles R, identiques ou différents, représentent :
      • un radical alkyle linéaire ou ramifié contenant 1 à 8 atomes de carbone, éventuellement substitué par au moins un halogène, de préférence le fluor, les radicaux alkyle étant de préférence méthyle, éthyle, propyle, octyle et 3,3,3-trifluoropropyle,
      • un radical cycloalkyle contenant entre 5 et 8 atomes de carbone cycliques, éventuellement substitué,
      • un radical aryle contenant entre 6 et 12 atomes de carbone pouvant être substitué, de préférence phényle ou dichlorophényle,
      • une partie aralkyle ayant une partie alkyle contenant entre 5 et 14 atomes de carbone et une partie aryle contenant entre 6 et 12 atomes de carbone, substituée éventuellement sur la partie aryle par des halogènes, des alkyles et/ou des alkoxyles contenant 1 à 3 atomes de carbone,
   • les symboles U, identiques ou différents, représentent (II) : 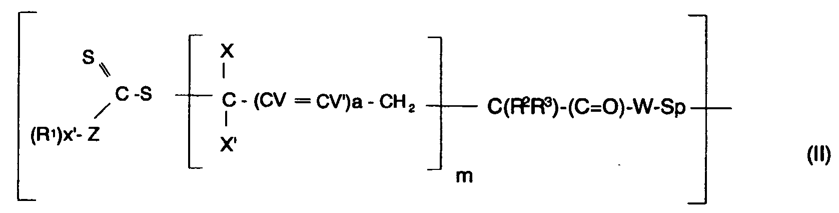
   dans lequel :
- x' = 1, 2,3 ou 4 en fonction de la valence de Z,
- Z, identiques ou différents, représentent un atome de carbone, soufre, oxygène, phosphore, azote et/ou une valence libre,
- R¹, identiques ou différents, représentent :
   - un groupe (i) alkyle, acyle, aryle, alcène ou alcyne éventuellement substitué,
   - un cycle (ii) carboné, saturé ou non, éventuellement substitué et/ou aromatique, et/ou
   - un hétérocycle (iii), saturé ou non, éventuellement substitué,
   - ces radicaux (i), (ii) et (iii) pouvant être substitués avantageusement par: des groupes phényles substitués, des groupes aromatiques substitués, ou des groupes : alkoxycarbonyle, aryloxycarbonyle (-COOR⁵), carboxy (-COOH), acyloxy (-O₂CR⁵), carbamoyle (-CONR⁵₂), cyano (-CN), alkylcarbonyle, alkylarylcarbonyle, arylcarbonyle, arylalkylcarbonyle, phtalimido, maleimido, succinimido, amidino, guanidimo, hydroxy (-OH), amino (-NR⁵₂), halogène, allyle, époxy, alkoxy (-OR⁵), S-alkyle, S-aryle, des groupes présentant un caractère hydrophile ou ionique tels que les sels alcalins d'acides carboxyliques, les sels alcalins d'acide sulfonique, les chaînes polyoxyde d'alkylène (POE, POP), les substituants cationiques (sels d'ammonium quaternaires), R⁵, identiques ou différents, représentant un groupe alkyle ou aryle, et/ou une chaîne polymère,
   - un groupement (iv) de formule -CₙF₍₂ₙ₊₁₎ avec n compris entre 1 et 20,
   - un groupement (v) de formule suivante : 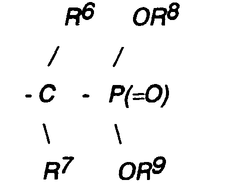 dans laquelle :
      - R⁶, R⁷, identiques ou non, sont choisis parmi un groupe halogène, -NO₂, -SO₃R¹⁰, -NCO, -CN, -OR¹⁰, -SR¹⁰, -N(R¹⁰)₂, -COOR¹⁰, -O₂CR¹⁰, -CON(R¹⁰)₂, -NCO(R¹⁰)₂ et -CₙF₍₂ₙ₊₁₎ avec n compris entre 1 et 20, de préférence égal à 1;
      - avec R¹⁰ représentant :
         * un atome d'hydrogène,
         * ou un radical alkyle, alcényle, alcynyle, cycloacényle, cycloalcynyle, alkaryle, aralkyle, hétéroaryle ou aryle éventuellement condensé à un hétérocycle aromatique ou non; ces radicaux pouvant éventuellement être substitués par :
            . un ou plusieurs groupes identiques ou différents, choisis parmi les atomes d'halogènes, =O, =S, -OH, alcoxy, SH, thioalcoxy, NH₂, mono ou di-alkylamino, CN, COOH, ester, amide, CₙF₍₂ₙ₊₁₎ et/ou éventuellement interrompu par un ou plusieurs atomes choisis parmi O, S, N, P ;
            . ou un groupe hétérocyclique éventuellement substitué par un ou plusieurs groupes tels que définis précédemment ;
            . ou R⁶ et R⁷ forment ensemble avec l'atome de carbone auquel ils sont attachés, un groupe =O, =S, un cycle hydrocarboné ou un hétérocycle ;
      - et R⁸ et R⁹, identiques ou différents, représentent un groupe tel que défini ci-dessus pour R¹⁰; ou forment ensemble une chaîne hydrocarbonée en C₂-C₄, éventuellement interrompue par un hétéroatome choisi parmi O, S et N ;
- V et V', identiques ou différents, représentent : H, un groupe alkyle ou un halogène,
- X et X', identiques ou différents, représentent H, un halogène ou un groupe R⁴, OR⁴, O₂COR⁴, NHCOH, OH, NH₂, NHR⁴, N(R⁴)₂, (R⁴)₂N⁺O⁻, NHCOR⁴, CO₂H, CO₂R⁴, CN, CONH₂, CONHR⁴ ou CONR⁴₂, dans lesquels R⁴ est choisi parmi les groupes alkyle, aryle, aralkyle, alkaryle, alcène ou organosilyle, éventuellement perfluorés et éventuellement substitués par un ou plusieurs groupes carboxyle, époxy, hydroxyle, alkoxy, amino, halogène ou sulfonique,
- R² et R³, identiques ou différents, représentent :
   - un groupe (i) alkyle, acyle, aryle, alcène ou alcyne éventuellement substitué,
   - un cycle (ii) carboné, saturé ou non, éventuellement substitué et/ou aromatique,
   - un hétérocycle (iii), saturé ou non, éventuellement substitué,
   - un atome d'hydrogène, des groupes : alkoxycarbonyle, aryloxycarbonyle (-COOR⁵), carboxy (-COOH), acyloxy (-O₂CR⁵), carbamoyle (-CONR⁵₂), cyano (-CN), alkylcarbonyle, alkylarylcarbonyle, arylcarbonyle, arylalkylcarbonyle, phtalimido, maleïmido, succinimido, amidino, guanidimo, hydroxy (-OH), amino (-NR⁵₂), halogène, allyle, époxy, alkoxy (-OR⁵), S-alkyle, S-aryle, des groupes présentant un caractère hydrophile ou ionique tels que les sels alcalins d'acides carboxyliques, les sels alcalins d'acide sulfonique, les chaînes polyoxyde d'alkylène (POE, POP), les substituants cationiques (sels d'ammonium quaternaires), R⁵, identiques ou différents, représentant un groupe alkyle ou aryle, et/ou une chaîne polymère,
   - les radicaux (i), (ii) et (iii) pouvant être substitués avantageusement par: des groupes phényles substitués, des groupes aromatiques substitués, ou des groupes : alkoxycarbonyle, aryloxycarbonyle (-COOR⁵), carboxy (-COOH), acyloxy (-O₂CR⁵), carbamoyle (-CONR⁵₂), cyano (-CN), alkylcarbonyle, alkylarylcarbonyle, arylcarbonyle, arylalkylcarbonyle, phtalimido, maleïmido, succinimido, amidino, guanidimo, hydroxy (-OH), amino (-NR⁵₂), halogène, allyle, époxy, alkoxy (-OR⁵), S-alkyle, S-aryle, des groupes présentant un caractère hydrophile ou ionique tels que les sels alcalins d'acides carboxyliques, les sels alcalins d'acide sulfonique, les chaînes polyoxyde d'alkylène (POE, POP), les substituants cationiques (sels d'ammonium quaternaires), R⁵, identiques ou différents, représentant un groupe alkyle ou aryle, et/ou une chaîne polymère,
- W, identiques ou différents, représentent un radical divalent choisi parmi -O-, -NR⁴-, -NH-, -S-,
- Sp, identiques ou différents, représentent une rotule constituée d'un radical divalent organique de formule -(CH₂)_{x"-} dans lequel x"' est compris entre 1 et 20, ce radical pouvant être substitué et/ou contenir au moins un hétéroatome,
- a = 0 ou 1,
- m ≥ 1, et lorsque m > 1 les motifs unitaires répétitifs d'indice m sont identiques ou différents,
procédé dans lequel on met en contact :
- au moins un monomère éthyléniquement insaturé de formule (III):

   CXX' (= CV - CV')ₐ = CH₂,
- un composé silicone précurseur comprenant des motifs, identiques ou différents de formule (IV):

   RₓU'_{y}SiO_{[4-(x+y)]/2}

   dans lequel :
- R, x et y correspondent aux valeurs données précédemment,
   et le radical monovalent U' est selon la formule suivante (V) : 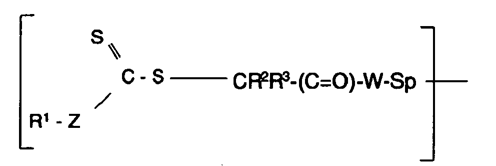
- et un initiateur de polymérisation radicalaire.

L'initiateur de polymérisation radicalaire peut être choisi parmi les initiateurs classiquement utilisés en polymérisation radicalaire. Il peut s'agir par exemple d'un des initiateurs suivants :
- les peroxydes d'hydrogène, tels que l'hydroperoxyde de butyle tertiaire, l'hydroperoxyde de cumène, le t-butyl-peroxyacétate, le t-butylperoxybenzoate, le t-butylperoxyoctoate, le t-butylperoxynéodécanoate, le t-butylperoxyisobutarate, le peroxyde de lauroyle, le t-amylperoxypivalte, le t-butylperoxypivalate, le peroxyde de dicumyl, le peroxyde de benzoyle, le persulfate de potassium, le persulfate d'ammonium,
- les composés azoïques tels que : le 2-2'-azobis(isobutyronitrile), le 2,2'-azobis(2-butanenitrile), le 4,4'-azobis(4-acide pentanoïque), le 1,1'-azobis(cyclohexane-carbonitrile), le 2-(t-butylazo)-2-cyanopropane, le 2,2'-azobis[2-méthyl-N-(1,1)-bis(hydroxyméthyl)-2-hydroxyéthyl] propionamide, le 2,2'-azobis(2-méthyl-N-hydroxyéthyl]-propionamide, le dichlorure de 2,2'-azobis(N,N'-diméthylène isobutyramidine), le dichlorure de 2,2'-azobis (2-amidinopropane), le 2,2'-azobis (N,N'-diméthylèneisobutyramide), le 2,2'-azobis(2-méthyl-N-[1,1-bis (hydroxyméthyl)-2-hydroxyéthyl] propionamide), le 2,2'-azobis(2-méthyl-N-[1,1-bis (hydroxyméthyl)éthyl] propionamide), le 2,2'-azobis[2-méthyl-N-(2-hydroxyéthyl) propionamide], le 2,2'-azobis(isobutyramide) dihydrate,
- les systèmes redox comportant des combinaisons telles que : les mélanges de peroxyde d'hydrogène, d'alkyle, peresters, percarbonates et similaires et de n'importe lequel des sels de fer, de sels titaneux, formaldéhyde sulfoxylate de zinc ou formaldéhyde sulfoxylate de sodium, et des sucres réducteurs ; les persulfates, perborate ou perchlorate de métaux alcalins ou d'ammonium en association avec un bisulfite de métal alcalin, tel que le métabisulfite de sodium, et des sucres réducteurs ; les persulfates de métal alcalin en association avec un acide arylphosphinique, tel que l'acide benzène phosphonique et autres similaires, et des sucres réducteurs.

La quantité d'initiateur à utiliser est déterminée de manière à ce que la quantité de radicaux générés soit d'au plus 20 % en mole par rapport à la quantité de composé précurseur silicone (IV), de préférence d'au plus 5 % en mole.

Comme monomère éthyléniquement insaturé, on utilise ceux qui ont été cités précédemment pour définir la fraction du copolymère soluble dans la phase organique non silicone.

Précisons en outre que le butadiène et le chloroprène correspondent au cas où a = 1 dans la formule (I) et (III),

Pour la préparation des copolymères hybrides de formule (I) pour lesquels X = H et X' = NH₂, on utilise de préférence à titre de monomères éthyléniquement insaturés les amides de la vinylamine, par exemple le vinylformamide ou le vinylacétamide. Puis le copolymère obtenu est hydrolysé à pH acide ou basique.

Pour la préparation des copolymères hybrides de formule (I) pour lesquels X = H et X' = OH, on utilise de préférence à titre de monomères éthyléniquement insaturés les esters vinyliques d'acide carboxylique, comme par exemple l'acétate de vinyle. Puis le copolymère obtenu est hydrolysé à pH acide ou basique.

Les types et quantités de monomères copolymérisables mis en oeuvre selon la présente invention varient en fonction de l'application finale particulière à laquelle est destiné le copolymère hybride.

Selon une première variante préférée, le copolymère hybride silicone et organique est constitué d'un squelette silicone linéaire comprenant de 1 à 300 motifs de formule (I), de préférence 1 à 200, et portant de 1 à 50 radicaux U, de préférence 1 à 10.

Selon une deuxième variante, au moins un des radicaux monovalents U' est de préférence de formule (VI) : 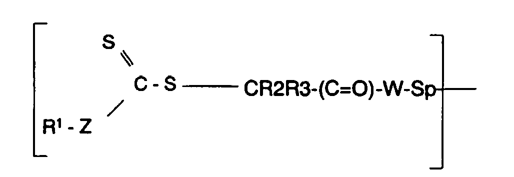 dans laquelle R² et R³, identiques ou différents, représentent un atome d'hydrogène, un groupe cyano, allkoxycarbonyle, alkyle, acyle, aryle, alcène ou alcyne éventuellement substitué; et W est de définition identique à celle donnée précédemment, et de préférence est égal à un atome d'oxygène. A titre d'exemples, on citera les précurseurs silicones dans lesquelles U' correspond à :

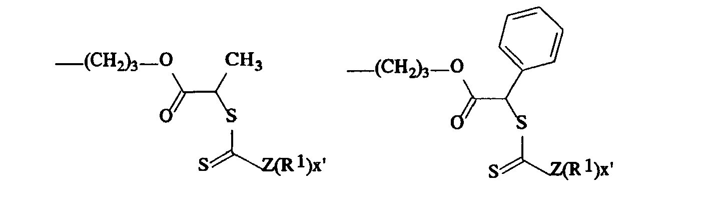

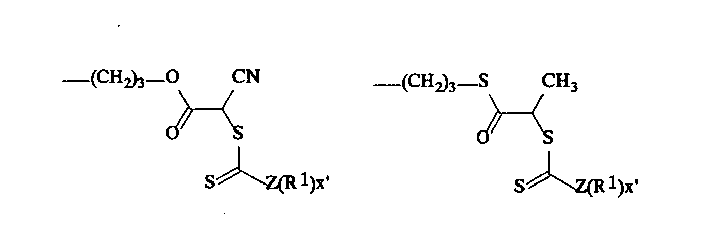

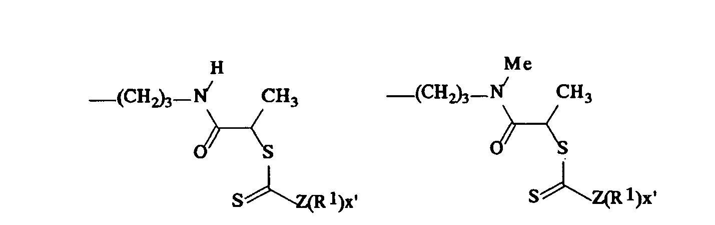

Selon une troisième variante de l'invention, au moins une partie des radicaux monovalents U' de(s) précurseur(s) silicone(s) (IV) et donc au moins une partie des groupements U du copolymère hybride obtenu sont tels que Z soit un atome d'oxygène et/ou un atome de soufre.

Selon une quatrième variante, outre des motifs de formule (I), le copolymère hybride silicone et organique selon l'invention peut comprendre des motifs RₓU_{y}F_{z}SiO_{[4}-_{(x+y+z)]/2} (XIV) dans lesquels:
- x est égal à 0, 1, 2 ou 3, y est égal à 0, 1, 2 ou 3 et z est égal à 1, 2 ou 3 avec avec 2 ≤ (x+y+z) ≤ 3,
- et F est un groupement porteur d'au moins une fonction tels que hydroxy, alkoxy, thiol, amine, époxy et/ou polyether.

Ces groupements F peuvent éventuellement apporter des propriétés complémentaires et/ou supplémentaires aux copolymères hybrides préparés selon le procédé de l'invention. Ils peuvent être notamment contenus initialement au sein du précurseur silicone de formule (IV).

Outre des copolymères hybrides avec des segments organiques homopolymères, le procédé qui vient d'être décrit permet de préparer des polymères hybrides porteurs de groupements organiques à blocs (c'est-à-dire multiblocs). Pour cela, le procédé consiste à répéter la mise en oeuvre du procédé de préparation précédemment décrit en utilisant :
- des monomères différents de la mise en oeuvre précédente, et
- à la place du composé silicone précurseur (II), le copolymère hybride comprenant des motifs (I) à blocs issu de la mise en oeuvre précédente.

Selon ce procédé de préparation de copolymères à blocs, lorsque l'on souhaite obtenir des copolymères à blocs homogènes et non à gradient de composition, et si toutes les polymérisations successives sont réalisées dans le même réacteur, il est essentiel que tous les monomères utilisés lors d'une étape aient été consommés avant que la polymérisation de l'étape suivante ne commence, donc avant que les nouveaux monomères ne soient introduits.

Comme pour le procédé de polymérisation de copolymère monobloc, ce procédé de polymérisation de copolymères à blocs présente l'avantage de conduire à des copolymères à blocs présentant un indice de polydispersité faible. Il permet également de contrôler la masse moléculaire des polymères à blocs.

Le composé silicone précurseur de formule générale (IV) utilisé au sein du procédé de préparation de copolymères hybrides selon l'invention peut être obtenu par réaction :
(i) d'une silicone comprenant des motifs de formule (VII) :

   RₓU"_{y}SiO_{[4-(x+y)]/2}

   où le radical monovalent U" est selon la formule suivante (VIII): 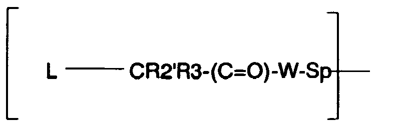 dans laquelle :
   - W et Sp sont de définitions identiques à celles données précédemment,
   - L est un groupement électrofuge, par exemples : Br⁻, Cl⁻, I⁻, OTs⁻, OMs⁻, (C₆H₆)-(C=O)-O⁻, (CH₃)-(C=O)-O⁻, (CF₃)-(C=O)-O⁻.
(ii) avec un composé choisi parmi ceux de formules générales suivantes (IX), (X) ou (XI) : 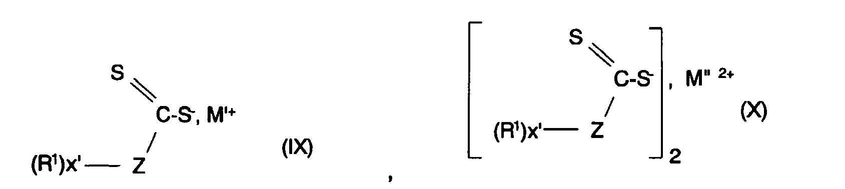 et 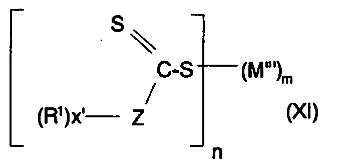 dans lesquelles :
   - M'⁺ représente K⁺, Na⁺, NR₄⁺, ou PR₄⁺' R étant de définition similaire à celle donnée pour R de la formule (I),
   - M"²⁺ représente un métal alcalino-terreux tels que Ca²⁺, Ba²⁺ et Sr²⁺⁺,
   - M'" représente Zn, Cd, m est égal à 1 ou 2, n est égal à 1, 2, 3 ou 4 et de préférence m est égal à 1 et n est égal à 2.

Cette silicone de formule (VII) peut être notamment obtenue à partir (i) d'une silicone comprenant des motifs de formule (XII) :RₓU"'_{y}SiO_{[4-(x+y)]/2} où le radical monovalent U"' est de formule (XIII) : -Sp-WH et (ii) d'un composé de formule :

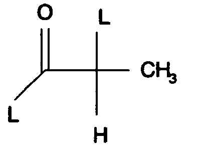

La polymérisation peut être réalisée en masse, en solution ou en émulsion. De préférence, elle est mise en oeuvre en émulsion.

De préférence, le procédé est mis en oeuvre de manière semi-continue.

La température peut varier entre la température ambiante et 150°C selon la nature des monomères utilisés.

En général, au cours de la polymérisation, la teneur instantanée en copolymère par rapport à la quantité instantanée en monomère et copolymère est comprise entre 50 et 99 % en poids, de préférence entre 75 et 99 %, encore plus préférentiellement entre 90 et 99 %. Cette teneur est maintenue, de manière connue, par contrôle de la température, de la vitesse d'addition des réactifs et de l'initiateur de polymérisation.

Enfin, le procédé est généralement mis en oeuvre en l'absence de source UV.

Il est à noter qu'il peut être avantageux de modifier chimiquement les extrémités xanthates du copolymère obtenu, en mettant en oeuvre toute méthode connue de l'homme de l'art, comme par exemple une étape d'hydrolyse.

Le procédé et les polymères obtenus par ce procédé sont décrits dans la demande française n° 00 09722 déposée le 25/07/00.

D'autres méthodes de synthèses de ce type de copolymère peuvent être mises en oeuvre, notamment celle décrite dans les demandes internationales WO 00/71606 et WO 00/71607.

Selon un mode de réalisation particulier de l'invention, la quantité de copolymère stabilisant interne représente de 0,5 à 10 % en poids de la phase organique interne, de préférence entre 1 et 4 % en poids par rapport à la même référence.

La proportion pondérale en phase organique interne par rapport à la phase organique, est plus particulièrement comprise entre 10/90 et 90/10, de préférence comprise entre 30/70 et 50/50.

Quant au rapport pondéral de l'ensemble (phase organique interne et phase organique) / phase aqueuse représente 0,1/99,9 à 90/10.

La phase aqueuse, dans laquelle est dispersée la phase organique comprenant la phase organique interne, comprend au moins un tensioactif et/ou polymère stabilisant externe, éventuellement au moins une matière active.

Ce qui a été détaillé dans le cadre de la variante précédente, reste valable ici et l'on pourra s'y référer.

L'émulsion simple ou l'émulsion phase aqueuse interne / phase organique ou l'émulsion phase organique interne /phase aqueuse est préparée en mettant en oeuvre les méthodes classiques.

Ainsi, dans le cas d'émulsions simples, on prépare d'une part, une premier mélange comprenant le composé utilisé comme phase organique, éventuellement une matière active, puis d'autre part, le mélange aqueux comprenant éventuellement la matière active, éventuellement le tensioactif / polymère amphiphile externe, et éventuellement le copolymère à blocs. On ajoute ensuite le mélange constituant la phase dispersée dans celui constituant la phase continue de l'émulsion.

Dans le cas d'émulsions multiples, on prépare tout d'abord l'émulsion "interne".

Par exemple, dans le cas d'une émulsion d'une phase aqueuse interne et d'une phase organique, on prépare deux mélanges, le premier, qui est un mélange aqueux comprenant éventuellement la matière active, éventuellement le tensioactif interne (s'il est plus particulièrement choisi parmi les tensioactifs cationiques), éventuellement au moins un additif (sel / sucre / polysaccharide), puis un second mélange comprenant le composé constituant la phase organique, éventuellement la matière active, éventuellement le tensioactif / polymère stabilisant interne ; puis on ajoute le premier mélange au second.

Dans le cadre d'une émulsion d'une phase organique interne dans une phase organique, on prépare là encore deux mélanges ; le premier constituant la phase organique interne comprenant le composé constituant la phase organique, éventuellement une matière active ; puis un second mélange comprenant le composé constituant ladite phase organique, éventuellement une matière active, et le polymère stabilisant interne ; le premier mélange est ensuite introduit dans le second.

Une fois l'émulsion interne préparée, on effectue un mise en émulsion de ladite émulsion, dans la phase aqueuse (qui sera la phase aqueuse externe de l'émulsion multiple).

Ainsi, le mélange constituant la phase aqueuse est préparé et comprend éventuellement une matière active, éventuellement un tensioactif / polymère amphiphile externe, éventuellement un additif (sel / sucre / polysaccharide), éventuellement le polymère épaississant, et éventuellement le copolymère à blocs.

En effet, selon une première possibilité, le copolymère à blocs est incorporé lors de la préparation même de l'émulsion. En d'autres termes on ajoute le copolymère à blocs lors de la préparation de l'émulsion de la phase organique et de la phase aqueuse ; la phase organique comprenant éventuellement une phase aqueuse interne dispersée ou une phase organique interne dispersée.

Plus précisément, ledit copolymère à blocs est présent dans le mélange constituant la phase aqueuse (phase aqueuse de l'émulsion simple ou phase aqueuse externe de l'émulsion multiple).

Il est indiqué que dans ce cas de figure, la présence d'un tensioactif et/ou polymère amphiphile externe peut ne pas être nécessaire, même si de manière avantageuse, ils sont présents dans la phase aqueuse.

Selon cette possibilité, le rapport pondéral phase organique (comprenant éventuellement la phase aqueuse ou organique interne) / phase aqueuse est de manière avantageuse compris entre 10/90 et 90/10.

De plus, dans le cas où un polymère épaississant est utilisé, il est préférable de l'incorporer une fois l'émulsion obtenue, de préférence sous la forme d'une solution aqueuse, pour laquelle la teneur en eau sera calculée de telle sorte que le rapport pondéral phase organique (comprenant éventuellement la phase aqueuse ou organique interne) / phase aqueuse soit compris dans la gamme 0,1/99,9 à 90/10.

Selon une deuxième possibilité, le copolymère à blocs est ajouté à l'émulsion, quelle que soit la variante concernée, une fois réalisée la préparation de l'émulsion même, c'est-à-dire la préparation de l'émulsion de la phase organique et de la phase aqueuse ; la phase organique comprenant éventuellement une phase aqueuse interne dispersée ou une phase organique interne dispersée.

Dans ce cas, le tensioactif et/ou polymère amphiphile externes est présent dans la phase aqueuse de l'émulsion simple ou multiple.

Dans le cadre de cette possibilité, ledit copolymère à blocs est ajouté à l'émulsion, avantageusement, sous la forme d'une solution aqueuse ; la quantité de phase aqueuse mise en oeuvre étant calculée en fonction de la quantité de phase aqueuse ajoutée lors de la préparation de l'émulsion, et de telle sorte que le rapport pondéral phase organique (comprenant éventuellement la phase aqueuse ou organique interne) / phase aqueuse soit compris dans la gamme 10/90 à 90/10.

Il est précisé de plus que le copolymère à blocs peut être introduit au moment de la préparation de la formulation aqueuse finale. En un tel cas, le rapport pondéral phase organique (comprenant éventuellement la phase aqueuse ou organique interne) / phase aqueuse est compris dans la gamme 0,1/99,9 à 10/90.

Ces opérations de mélanges lors des diverses étapes de la préparation de l'émulsion simple ou multiple, sont de manière habituelle réalisées sous agitation.

Par ailleurs, de manière classique, la préparation de l'émulsion est en général réalisée à une température supérieure ou égale à la température de fusion de la ou des phases organiques présentes. A titre indicatif, la température de préparation de l'émulsion est comprise entre 20 et 80°C.

Les émulsions qui viennent d'être détaillées peuvent être notamment utilisées dans des domaines du traitement de la peau et/ou du cheveu, du traitement des textiles (notamment pour le lavage des textiles), du traitement des plantes (domaine phytosanitaire), le traitement du métal.

L'un des intérêts des émulsions comprenant le copolymère à blocs dans des formulations aqueuses comprenant des tensioactifs anioniques (shampooing, gels douches, formulations de lavage des textiles etc.) est qu'elles permettent de déposer une émulsion sur une surface, en mettant en oeuvre des mécanismes de floculation par précipitation lors de la dilution de la formulation.

Un mode de réalisation particulièrement intéressant de l'invention, consiste en ce que :
- l'émulsion à déposer représente un élément constitutif d'une formulation détergente liquide aqueuse pour le lavage d'articles en fibres textiles, comprenant au moins un agent tensioactif anionique,
- l'émulsion est une émulsion simple, dont la phase organique comprend au moins une matière active hydrophobe organique ou organosilicique
- l'émulsion est destinée à se déposer à la surface des articles en fibres textiles au cours d'une opération de lavage.

Un objet particulièrement intéressant de l'invention vise donc un mode particulier de réalisation de celle-ci, consistant en l'utilisation, dans une formulation détergente liquide aqueuse pour le lavage des articles en fibres textiles, comprenant au moins un agent tensioactif anionique et au moins une matière active organique ou organosilicique hydrophobe en émulsion dans une phase aqueuse, d'au moins un copolymère à blocs comprenant au moins un bloc hydrophobe non ionique et au moins un bloc cationique dans les conditions de l'opération de lavage desdits articles, comme agent aidant au dépôt, au cours de l'opération de lavage, de ladite matière active en émulsion à la surface desdits articles.

Un autre objet de l'invention, vise un procédé pour aider, lors d'une opération de lavage d'articles en fibres textiles à l'aide d'une formulation détergente liquide aqueuse comprenant au moins un agent tensioactif anionique et au moins une matière active organique ou organosilicique hydrophobe en émulsion dans une phase aqueuse, le dépôt de ladite matière active en émulsion à la surface desdits articles, procédé consistant à ajouter à ladite formulation détergente au moins un copolymère à blocs comprenant au moins un bloc hydrophobe non ionique et au moins un bloc cationique dans les conditions de l'opération de lavage.

De manière préférentielle, la composition détergente liquide aqueuse pour le lavage des articles en fibres textiles peut comprendre de 5 à 50% en masse d'au moins un agent tensioactif anionique ; la quantité de matière active organique ou organosilicique hydrophobe peut représenter de 0,01 à 10%, de préférence de 0,1 à 5%, plus préférentiellement de 0,2 à 4% de la masse de la formulation ; la quantité de copolymère à blocs peut représenter de 0,1 à 25%, de préférence de 1 à 15% et plus particulièrement de 3 à 11 % de la masse de la matière active organique ou organosilicique hydrophobe.

La quantité de formulation détergente pouvant être mise en oeuvre pour réaliser l'opération de lavage peut être de l'ordre de 1 à 10 grammes par litre d'eau de lavage ; la matière active en émulsion flocule par précipitation notamment à la dilution.

La matière active organique ou organosilicique en émulsion est de préférence choisie parmi les huiles ou les cires d'origine végétales, les esters d'acides gras, les huiles minérales, les cires paraffiniques, les huiles ou cires silicones non-ioniques, les huiles silicones aminées, en particulier des huiles silicones à fonction(s) piperidinyle(s) encombrées. Ce type de matière active permet d'apporter aux surfaces textiles des propriétés d'antifroissage et/ou d'aide au repassage et/ou antisalissure et/ou de meilleure résistance à l'abrasion.

La matière active présente dans la phase organique de l'émulsion, peut en outre être présente en même temps qu'un autre constituant hydrophobe de détergence soluble dans ladite matière active ; on peut mentionner notamment les parfums, les azurants optiques, les agents biocides hydrophobes.

La formulation détergente dont l'émulsion de matière active constitue un des éléments essentiels, peut en outre contenir à côté du ou des agents tensioactifs anioniques, d'autres additifs usuellement mis en oeuvre dans les compositions détergentes, liquides notamment, pour le lavage des articles en fibres textiles.
On peut mentionner notamment des agents tensioactifs non-ioniques notamment, des adjuvants de détergence (« builder ») inorganiques (polyphopsphates, silicates, carbonates, zéolites ...) ou organiques (polyphosphonates hydrosolubles, polycarboxylates, polyacides polycarboxyliques, sels d'acides polyacétiques...), des agents antisalissures (dérivés cellulosiques, copolymères polyesters à base de motifs éthylène téréphtalate et polyoxyéthylène téréphtalate, oligomères ou copolymères polyesters sulfonés ...), des agents antiredéposition (monoamines ou polyamines éthoxylées, carboxyméthylcelluloses, polyvinylpyrrolidones ...), des agents chélatants du fer ou du magnésium (aminocarboxylates, aminophosphonates, dihydroxydisulfobenzène ...), des agents dispersants polymériques (sels hydrosolubles d'acides polycarboxyliques de masse moléculaire de l'ordre de 2000 à 100 000 g/mol, polyéthylènes glycols de masse moléculaire de l'ordre de 5000 à 75 000 g/mol ...), des agents de fluorescence, des agents supresseurs de mousses, des agents adoucissants (argiles ...) des enzymes, des agents tampons, des parfums, des pigments ...

Lorsque les émulsions comprenant le copolymère à blocs sont utilisés dans des formulations exemptes de tensioactifs anioniques, le dépôt desdites émulsions peut se faire au moyen d'interactions électrostatiques sur des surfaces présentant globalement un potentiel de charges anioniques

Dans le cas où les émulsions sont utilisées dans des compositions destinées au traitement de la peau et/ ou du cheveu, elles peuvent être introduites dans des formulations aqueuses destinées à être rincées et comprenant notamment de 3 à 50 % en poids de tensioactifs ioniques ou non ioniques.

Ces formulations comprennent les additifs classiques dans le domaine, comme des filtres UV, des agents dispersants, séquestrants, émollients, humectants, des conditionneurs, des résines fixatives, des agents plastifiants, des colorants, des pigments, des parfums, des agents bactéricides, etc.

Les exemples suivants sont donnés à titre illustratif.

### Exemple 1

### Synthèse d'un copolymère dibloc Polyacrylate de butyle 2000-Polyacrylate de 2-diméthylaminoéthyle 4000.

Dans un ballon bicol en verre de 1 litre muni d'un réfrigérant et d'une agitation magnétique et maintenu sous argon, on introduit 155,7 g d'éthanol, 10,41 g de *O*-ethyl-*S*-(1-methoxycarbonyl)ethyl) xanthate (CH₃CHCO₂CH₃)S(C=S)OEt et 100 g d'acrylate de butyle. La solution est mise en température à 70°C, et une solution de 3,28 g d'azobisisobutyronitrile (AIBN) dans 9,85 g d'acétone et 4,92 g d'éthanol est rajoutée. Trois heures après cet ajout, une solution de 1,64 g d'AIBN dans 4,93 g d'acétone et 2,46 g d'éthanol est additionnée.

A ce stade, un échantillon est prélevé. La masse molaire moyenne en nombre (Mn) est mesurée par chromatographie d'exclusion stérique dans le diméthylformamide, couplée à un détecteur de diffusion de lumière multi-angles (GPC-MALLS).
Mn=2200 g/mol.
Une solution de 200 g d'acrylate de 2-diméthylaminoéthyle dans 280 g d'éthanol est ajoutée pendant trois heures au milieu réactionnel. Deux heures après la fin de cette introduction, on ajoute une solution de 2,46 g d'AIBN dans 7,39 g d'acétone et 3,69 g d'éthanol. Deux heures plus tard, cette même solution d'AIBN est à nouveau ajoutée. Suite à ce dernier ajout, la réaction est poursuivie pendant deux heures, puis stoppée en refroidissant le milieu réactionnel à température ambiante.

### Exemple 2

### Synthèse d'un copolymère dibloc Polyacrylate de 2-triméthylaminoéthyle méthyl sulfate 40000 (cationique)-Polyacrylate de butyle 3000.

Une solution composée de 236 g d'eau, 75 g d'éthanol, et 308 g d'une solution d'acrylate de 2-triméthylaminoéthyle méthyl sulfate (i.e. acrylate de 2-diméthylaminoéthyle quaternisé au méthyl sulfate) à 80% dans l'eau est préparée dans un bécher. Dans un ballon bicol en verre de 1 litre muni d'un réfrigérant et d'une agitation magnétique et maintenu sous argon, on introduit 150 g de la solution décrite ci-dessus, et une solution de 1,28 g de *O*-ethyl-*S*-(1-methoxycarbonyl)ethyl) xanthate (CH₃CHCO₂CH₃)S(C=S)OEt dans 25 g d'éthanol. 0,834 g de 4,4'-azobis(4-cyanolvaleric acid) (ACP) sont également ajoutés au mélange réactionnel. La solution est mise en température à 70°C. 15 minutes après le début de la réaction, le reste de la solution de monomère est ajoutée en continu, sur une durée de 2,5 heures. En fin d'introduction de la solution de monomère, 0,85 g d'amorceur dans 5 g d'eau sont rajoutés au mélange réactionnel. La réaction est arrêtée après un temps global de polymérisation de 6 heures.
Un échantillon de 18 g de solution est alors prélevé. Une analyse RMN1 H confirme que le monomère acrylate a été totalement polymérisé.
Au polymère issu de la première étape, on additionne 18 g d'acrylate de butyle en une fois, suivis de 0,9 g d'ACP. Après 3 heures de réaction, 0,9 g d'ACP sont à nouveau ajoutés. La réaction est alors poursuivie pendant 5 heures. En fin de réaction, le copolymère dibloc est obtenu. Par simple évaporation de l'éthanol à l'évaporateur rotatif, une dispersion aqueuse stable du dibloc est obtenue.

### Exemple 3

On prépare, selon un mode opératoire analogue à celui de l'exemple 2, un copolymère dibloc Polyacrylate de 2-triméthylaminoéthyle méthyl sulfate 4000 (cationique)-Polyacrylate de butyle 3000.

### Exemple 4

### Préparation de l'émulsion A et du shampoing SA

On prépare 27 g d'une solution aqueuse contenant 0,3g en poids du copolymère à blocs de l'exemple 1. Le pH de cette solution est ajusté à 5. On y introduit alors 3 grammes d'huile silicone Rhodorsil 48V5000 de Rhodia (huile polydiméthylsiloxane bloquée à chacune des extrêmités par un motif (CH₃)₂ HO Si O_{0,5} et ayant une viscosité de 5000 mPa.s à 25°C). Le mélange est homogénéisé par un premier passage à l'ultra-turrax à 9500rpm pendant 5 minutes puis au microfluidiseur (5 fois à 500 bars). On obtient l'émulsion A.
On prépare 70 g d'une formulation aqueuse contenant 14g de lauryléthersulfate de sodium (Empicol ESB3M), 2g de tégobétaïne T7 et 1,6g de chlorure de sodium. Le pH est ajusté à 5. Les 30 g d'émulsion A sont alors introduits dans les 70 g de la formulation précédente et ce, sous agitation à l'aide d'une pale cadre (300tr/min pendant 5 minutes). On obtient le shampoing SA.

### Préparation de l'émulsion B et du shampoing SB comparatif

On prépare 27 g d'une solution aqueuse contenant 0,3g en poids sodium dodécylsulfate. Le pH de cette solution est ajusté à 5. On y introduit alors 3 grammes d'huile silicone Rhodorsil 48V5000 de Rhodia. Le mélange est homogénéisé par un premier passage à l'ultra-turrax à 9500rpm pendant 5 minutes puis au microfluidiseur (5 fois à 500 bars). On obtient l'émulsion B.
On prépare 70 g d'une formulation aqueuse contenant 14g de lauryléthersulfate de sodium (Empicol ESB3M), 2g de tégobétaïne T7 et 1,6g de chlorure de sodium. Le pH est ajusté à 5. Les 30 g d'émulsion B sont alors introduits dans les 70 g de la formulation précédente et ce, sous agitation à l'aide d'une pale cadre (300tr/min pendant 5 minutes). On obtient le shampoing SB.

### Comportement à la dilution des shampoings A et B

Les shampoings SA et SB sont observés par microscopie optique tels quels et après avoir été dilués 10 fois par une solution aqueuse à pH 5.
On observe clairement une floculation du shampoing SA à la dilution.
On n'observe en revanche aucune floculation du shampoing SB à la dilution.

### Déposition

Les shampoings SA et SB sont utilisés pour traiter des mèches de cheveux identiques. On mesure par fluorescence X le dépôt de silicone sur cheveu après lavage et rinçage des mèches. On constate que le dépôt de silicone obtenu avec le shampoing SA est supérieur à celui obtenu avec le shampoing SB.

### Exemple 5

### Préparation de l'émulsion C et du shampoing SC comparatif

On prépare 27 g d'une solution aqueuse contenant 0,3g en poids de sodium dodécylsulfate. Le pH de cette solution est ajusté à 5.
On y introduit alors 3 grammes d'huile silicone Rhodorsil 48V5000 de Rhodia. Le mélange est homogénéisé par un premier passage à l'ultra-turrax à 9500rpm pendant 5 minutes puis au microfluidiseur (5 fois à 500 bars). On obtient l'émulsion C.
On prépare 70 g d'une formulation aqueuse contenant 14g de lauryléthersulfate de sodium (Empicol ESB3M), 2g de tégobétaïne T7 et 1,6g de chlorure de sodium. Le pH est ajusté à 5. Les 30 g d'émulsion C sont alors introduits dans les 70 g de la formulation précédente et ce, sous agitation à l'aide d'une pale cadre (300tr/min pendant 5 minutes). On obtient le shampoing SC.

### Préparation du shampoing SD

On prépare 69,7 g d'une formulation aqueuse contenant 14g de lauryléthersulfate de sodium (Empicol ESB3M), 2g de tégobétaïne T7 et 1,6g de chlorure de sodium. Le pH est ajusté à 5. Les 30 g d'émulsion C sont alors introduits dans les 69,7 g de la formulation précédente et ce, sous agitation à l'aide d'une pale cadre (300tr/min pendant 5 minutes). On introduit alors sous agitation à l'aide d'une pale cadre (300tr/min pendant 2 minutes), 0,3 g de copolymère à blocs de l'exemple 2. On obtient le shampoing SD.

### Déposition

Les shampoings SC et SD sont utilisés pour traiter des mèches de cheveux identiques. On mesure par fluorescence X le dépôt de silicone sur cheveu après lavage et rinçage des mèches. On constate que le dépôt de silicone obtenu avec le shampoing SD est supérieur à celui obtenu avec le shampoing SC.

### Exemple 6

On prépare deux émulsions aqueuses E1 et E2 d'huile silicone à partir de
• 10 parties en masse d'huile silicone Rhodorsil 47V100 de Rhodia (huile polydiméthylsiloxane bloquée à chacune des extrémités par un motif (CH₃)₃ Si O_{0,5} et ayant une viscosité de 100 mPa.s à 25°C)
• 100 parties d'eau
• et respectivement 0,5 partie (E1) et 1 partie (E2) en masse de copolymère à blocs de l'exemple 3
par passage du mélange pendant 5 minutes dans un homogénéiseur ultra-turrax tournant à 9500 tours par minute.
On obtient une émulsion dont la taille est respectivement de 0,5µm (E1) et 0,3µm (E2).

A titre comparatif on prépare une émulsion E' d'huile silicone à partir de
• 10 parties en masse d'huile silicone Rhodorsil 47V100
• 100 parties d'eau
• et 1 partie en masse de tensioactifs non-ionique Symperonic A7 (alcool gras ethoxylé)
par passage du mélange pendant 5 minutes dans un homogénéiseur ultra-turrax tournant à 9500 tours par minute.
On obtient une émulsion dont la taille est de 0,25 µm.

Ces trois émulsions, à raison de 1 partie en masse d'émulsion, exprimée en huile silicone, sont additionnées à 100 parties en masse d'une lessive liquide anionique L dont la composition est la suivante

| **Constituants** | **% en masse** |
|---|---|
| Sodium carbonate | 20% |
| Lauryl Sulfonate | 15% |
| Tesioactif non-ionique en C12 avec 7 OE | 10% |
| Sokalan CP5 de BASF (copolymère acide acrylique /anhydride maleïque, sel de sodium) | 4% |
| HEDP (séquestrant phosphonaté) | 0,5% |
| Anti-mousse | 0,2% |
| Enzyme (mélange de protease, amylase, mannanase, lipase, cellulase) | 0,5% |
| Parfum | 0, 5% |
| Propylene Glycol | 5% |
| Eau | Qsp 100 |

On obtient les lessives L1, L2 et L', correspondant respectivement à l'addition des émulsions E1, E2 et E' à la lessive L.

On réalise des opérations de lavage à l'aide des différentes lessives L, L', L1 et L2, dans un appareil de laboratoire Tergotomètre bien connu dans la profession des formulateurs de compositions détergentes. L'appareil simule les effets mécaniques et thermiques des machines à laver de type américain à pulsateur, mais grâce à la présence de 6 pots de lavage, il permet de réaliser des séries d'essais simultanés avec une économie de temps appréciable.
Pour chaque opération de lavage, on découpe deux éprouvettes de coton serviette éponge et deux éprouvettes de coton plat de dimensions 10x10 cm.
Elles sont lavées à l'aide de la formulation détergente choisie et rincées 3 fois, dans les conditions suivantes :
- nombre d'éprouvettes par pot du Tergotomètre : 3
- volume d'eau : 1 litre
- eau de dureté française 15°TH (eau du robinet)
- concentration en lessive choisie : 5 ml/l
- température de lavage : 40°C
- durée du lavage : 20 min
- vitesse d'agitation du Tergotomètre 100 tours/minute
- rinçage à l'eau froide (environ 15°TH)
- durée de chaque rinçage : 5 minutes
Les éprouvettes sont mises à sécher verticalement à température ambiante.

Dix experts son chargés d'effectuer un test manuel de douceur des échantillons en coton serviette éponge lavés et donner à chaque échantillon testé une note allant de 1 à 5.
La note 1 est donnée à un tissu très rêche
La note 5 est donnée à un tissu très doux (état neuf)
Pour chaque échantillon testé on calcule la moyenne des valeurs obtenues, ainsi que la moyenne des écarts types.
Les résultats obtenus sont les suivants :

| **Lessive** | **L** | **L'** | **L1** | **L2** |
|---|---|---|---|---|
| **Douceur** | 1,4 ± 0,51 | 1,9 ± 0,73 | 3,8 ± 0,63 | 4,3 ± 0,67 |

On constate que le copolymère à blocs de l'exemple 3 est particulièrement bien adapté à l'amélioration du dépôt de l'huile silicone en émulsion à la surface du coton, se concrétisant par un plus grand apport de douceur.

## Revendications

1. Utilisation comme aide au dépôt d'une émulsion sur une surface, d'au moins un copolymère à blocs comprenant au moins un bloc hydrophobe non ionique et au moins un bloc à motifs cationiques dans les conditions d'utilisation de ladite émulsion ; ladite émulsion comprenant une phase organique dispersée dans une phase aqueuse et comprenant éventuellement au moins un tensioactif et/ou au moins un polymère amphiphile externe(s).

2. Procédé pour aider le dépôt d'une émulsion sur une surface, dans les conditions d'utilisation de ladite émulsion, par addition à ladite émulsion d'au moins un copolymère à blocs comprenant au moins un bloc hydrophobe non ionique et au moins un bloc à motifs cationiques dans les conditions d'utilisation de ladite émulsion ; ladite émulsion comprenant une phase organique dispersée dans une phase aqueuse et comprenant éventuellement au moins un tensioactif et/ou au moins un polymère amphiphile externe(s).

3. Utilisation selon la revendication 1 ou procédé selon la revendication 2, **caractérisé**(e) en ce que le bloc hydrophobe non ionique du copolymère à bloc est obtenu à partir d'au moins un monomère choisi parmi :
- les esters des acides mono- ou poly- carboxyliques, linéaires, ramifiés, cycliques ou aromatiques, comprenant au moins une insaturation éthylénique ;
- les esters d'acides carboxyliques saturés comprenant 8 à 30 atomes de carbone, éventuellement porteurs d'un groupement hydroxyle ;
- les nitriles αβ-éthyléniquement insaturés, les éthers vinyliques, les esters vinyliques, les monomères vinylaromatiques, les halogénures de vinyle ou de vinylidène ;
- les monomères hydrocarbonés, linéaires ou ramifiés, aromatiques ou non, comprenant au moins une insaturation éthylénique ;
- l'oxyde de propylène, l'oxyde de butylène ;
seuls ou en mélanges, ainsi que les macromonomères dérivant de tels monomères dérivant de tels monomères.

4. Utilisation selon la revendication 1 ou 3 ou procédé selon la revendication 2 ou 3, **caractérisé**(e) en ce que le bloc à motifs cationiques du copolymère à bloc est obtenu à partir d'au moins un monomère choisi parmi :
- les (méth)acrylates d'aminoalkyle, les (méth)acrylamides d'aminoalkyle ;
- les monomères comprenant au moins une fonction amine secondaire, tertiaire ou quaternaire, ou un groupe hétérocyclique contenant un atome d'azote, la vinylamine, l'éthylène imine ;
- les sels d'ammonium de diallyldialkyl ;
seuls ou en mélanges, les sels correspondants, ainsi que les macromonomères dérivant de tels monomères.

5. Utilisation selon l'une quelconque des revendications 1, 3 ou 4 ou procédé selon l'une quelconque des revendications 2 à 4, **caractérisé**(e) en ce que l'un et/ou l'autre des deux blocs comprennent un ou plusieurs monomères hydrophiles non ioniques, choisis parmi, seuls ou en mélange, ou sous la forme de macromonomères, l'oxyde d'éthylène ; les amides des acides mono- ou poly- carboxyliques, linéaires, ramifiés, cycliques ou aromatiques, comprenant au moins une insaturation éthylénique ou dérivés ; les esters hydrophiles dérivant de l'acide (méth)acrylique ; les esters vinyliques permettant d'obtenir des blocs alcool polyvinylique après hydrolyse ; les monomères de type sucres.

6. Utilisation selon l'une quelconque des revendications 1 ou 3 à 5 ou procédé selon l'une quelconque des revendications 2 à 5, **caractérisé**(e) en ce que le copolymère à blocs est un copolymère linéaire comprenant deux blocs.

7. Utilisation selon l'une quelconque des revendications 1 ou 3 à 5 ou procédé selon l'une quelconque des revendications 2 à 5, **caractérisé**(e) en ce que le copolymère à blocs est un copolymère peigne ou greffé comprenant un squelette non ionique, de préférence hydrophile, comprenant des segments pendants cationiques et hydrophobes non ioniques.

8. Utilisation selon l'une quelconque des revendications 1 ou 3 à 5 ou procédé selon l'une quelconque des revendications 2 à 5, **caractérisé**(e) en ce que le copolymère à blocs est un copolymère de structure étoile.

9. Utilisation selon l'une quelconque des revendications 1 ou 3 à 8 ou procédé selon l'une quelconque des revendications 2 à 8, **caractérisé**(e) en ce que la masse molaire en poids du copolymère à blocs est comprise entre 2000 et 100000 g/mol, de préférence entre 2000 et 20000 g/mol.

10. Utilisation selon l'une quelconque des revendications 1 ou 3 à 9 ou procédé selon l'une quelconque des revendications 2 à 9, **caractérisé**(e) en ce que le copolymère à blocs est tel que la masse molaire en poids des blocs à motifs cationiques représente au moins deux fois la masse des blocs hydrophobes non ioniques, de préférence au moins cinq fois.

11. Utilisation selon l'une quelconque des revendications 1 ou 3 à 10 ou procédé selon l'une quelconque des revendications 2 à 10, **caractérisé**(e) en ce que le ou les blocs cationiques du copolymère à blocs sont tels qu'au moins 5 % en moles des monomères à partir desquels le(s) bloc(s) sont obtenus, sont des monomères cationiques, de préférence au moins 30 % en moles.

12. Utilisation selon l'une quelconque des revendications 1 ou 3 à 11 ou procédé selon l'une quelconque des revendications 2 à 11, **caractérisé**(e) en ce que le ou les blocs à motifs cationiques du copolymère à blocs sont obtenus à partir de monomères cationiques, et de préférence le ou les blocs cationiques sont des homopolymères.

13. Utilisation selon l'une quelconque des revendications 1 ou 3 à 12 ou procédé selon l'une quelconque des revendications 2 à 12, **caractérisé**(e) en ce que la phase organique de l'émulsion comprend une phase aqueuse interne dispersée dans ladite phase organique ; l'ensemble de la phase organique et de la phase aqueuse interne comprenant au moins un tensioactif et/ou au moins un polymère amphiphile internes.

14. Utilisation selon l'une quelconque des revendications 1 ou 3 à 9 ou procédé selon l'une quelconque des revendications 2 à 9, **caractérisé**(e) en ce que la phase organique de l'émulsion comprend une phase organique interne dispersée dans ladite phase organique ; l'ensemble de la phase organique et de la phase organique interne comprenant au moins un polymère stabilisant interne.

15. Utilisation selon l'une quelconque des revendications 1 ou 3 à 14 ou procédé selon l'une quelconque des revendications 2 à 14, **caractérisé**(e) en ce que la teneur en copolymère à blocs représente 0,1 à 30 % en poids par rapport au poids de phase organique.

16. Utilisation selon l'une quelconque des revendications 1 ou 3 à 15 ou procédé selon l'une quelconque des revendications 2 à 15, **caractérisé**(e) en ce que l'on ajoute le copolymère à blocs lors de la préparation de l'émulsion de la phase organique et de la phase aqueuse ; la phase organique comprenant éventuellement une phase aqueuse interne dispersée ou une phase organique interne dispersée.

17. Utilisation selon l'une quelconque des revendications 1 ou 3 à 16 ou procédé selon l'une quelconque des revendications 2 à 16, **caractérisé**(e) en ce que l'on ajoute le copolymère à blocs une fois réalisée la préparation de l'émulsion de la phase organique et de la phase aqueuse ; la phase organique comprenant éventuellement une phase aqueuse interne dispersée ou une phase organique interne dispersée.

18. Utilisation selon l'une quelconque des revendications 1 ou 3 à 17 ou procédé selon l'une quelconque des revendications 2 à 17, **caractérisé**(e) en ce que l'émulsion est un élément constitutif d'une formulation destinée au traitement de la peau et/ou du cheveu, des textiles, le domaine phytosanitaire, le traitement du métal.

19. Utilisation ou procédé selon la revendication 18, **caractérisé**(e) en ce que:
- l'émulsion à déposer représente un élément constitutif d'une formulation détergente liquide aqueuse pour le lavage d'articles en fibres textiles, comprenant au moins un agent tensioactif anionique,
- l'émulsion est une émulsion simple, dont la phase organique comprend au moins une matière active hydrophobe organique ou organosilicique
- l'émulsion est destinée à se déposer à la surface des articles en fibres textiles au cours d'une opération de lavage.

20. Utilisation ou procédé selon la revendication 19, **caractérisé**(e) en ce que la matière active hydrophobe organique ou organosilicique est choisie parmi les huiles ou les cires d'origine végétales, les esters d'acides gras, les huiles minérales, les cires paraffiniques, les huiles ou cires silicones non-ioniques, les huiles silicones aminées, en particulier des huiles silicones à fonction(s) piperidinyle(s) encombrées.

21. Utilisation ou procédé selon la revendication 20, **caractérisé**(e) en ce que la matière active hydrophobe organique ou organosilicique en émulsion apporte aux surfaces textiles sur lesquelles elle est déposée des propriétés d'antifroissage et/ou d'aide au repassage et/ou antisalissure et/ou de meilleure résistance à l'abrasion.

## Claims

1. The use, to assist in the deposition of an emulsion on a surface, of at least one block copolymer comprising at least one nonionic hydrophobic block and at least one block containing cationic units, under the conditions of use of said emulsion; said emulsion comprising an organic phase dispersed in an aqueous phase and optionally comprising at least one outer surfactant and/or at least one outer amphiphilic polymer.

2. A process to assist the deposition of an emulsion on a surface, under the conditions of use of said emulsion, by adding to said emulsion at least one block copolymer comprising at least one nonionic hydrophobic block and at least one block containing cationic units under the conditions of use of said emulsion; said emulsion comprising an organic phase dispersed in an aqueous phase and optionally comprising at least one outer surfactant and/or at least one outer amphiphilic polymer.

3. The use as claimed in claim 1 or the process as claimed in claim 2, **characterized in that** the nonionic hydrophobic block of the block copolymer is obtained from at least one monomer chosen from:
- linear, branched, cyclic or aromatic monocarboxylic or polycarboxylic acid esters, comprising at least one ethylenic unsaturation;
- saturated carboxylic acid esters comprising 8 to 30 carbon atoms, optionally bearing a hydroxyl group;
- α,β-ethylenically unsaturated nitriles, vinyl ethers, vinyl esters, vinylaromatic monomers, vinyl halides or vinylidene halides;
- linear or branched, aromatic or non-aromatic hydrocarbon-based monomers comprising at least one ethylenic unsaturation;
- propylene oxide or butylene oxide;
alone or as blends, and also the macromonomers derived from such monomers.

4. The use as claimed in claim 1 or 3 or the process as claimed in claim 2 or 3, **characterized in that** the block containing cationic units of the block copolymer is obtained from at least one monomer chosen from:
- aminoalkyl (meth)acrylates and aminoalkyl(meth)-acrylamides;
- monomers comprising at least one secondary, tertiary or quaternary amine function, or a heterocyclic group containing a nitrogen atom, vinylamine or ethyleneimine;
- diallyldialkylammonium salts;
alone or as blends, or the corresponding salts, and also the macromonomers derived from such monomers.

5. The use as claimed in any one of claims 1, 3 and 4 or the process as claimed in any one of claims 2 to 4, **characterized in that** either and/or both of the two blocks comprise one or more nonionic hydrophilic monomers chosen from, alone or as a mixture, or in the form of macromonomers, ethylene oxide; linear, branched, cyclic or aromatic monocarboxylic or polycarboxylic acid amides comprising at least one ethylenic unsaturation or derivatives thereof; hydrophilic esters derived from (meth)acrylic acid; vinyl esters for obtaining polyvinyl alcohol blocks after hydrolysis; monomers of sugar type.

6. The use as claimed in any one of claims 1 and 3 to 5 or the process as claimed in any one of claims 2 to 5, **characterized in that** the block copolymer is a linear copolymer comprising two blocks.

7. The use as claimed in any one of claims 1 and 3 to 5 or the process as claimed in any one of claims 2 to 5, **characterized in that** the block copolymer is a comb or grafted copolymer comprising a nonionic skeleton, which is preferably hydrophilic, comprising cationic pendent segments and nonionic hydrophobic segments.

8. The use as claimed in any one of claims 1 and 3 to 5 or the process as claimed in any one of claims 2 to 5, **characterized in that** the block copolymer is a copolymer of starburst structure.

9. The use as claimed in any one of claims 1 and 3 to 8 or the process as claimed in any one of claims 2 to 8, **characterized in that** the weight-average molar mass of the block copolymer is between 2000 and 100 000 g/mol and preferably between 2000 and 20 000 g/mol.

10. The use as claimed in any one of claims 1 and 3 to 9 or the process as claimed in any one of claims 2 to 9, **characterized in that** the block copolymer is such that the weight-average molar mass of the blocks containing cationic units represents at least twice and preferably at least five times the mass of the nonionic hydrophobic blocks.

11. The use as claimed in any one of claims 1 and 3 to 10 or the process as claimed in any one of claims 2 to 10, **characterized in that** the cationic block(s) of the block copolymer is (are) such that at least 5 mol% and preferably at least 30 mol% of the monomers from which the block(s) is (are) obtained are cationic monomers.

12. The use as claimed in any one of claims 1 and 3 to 11 or the process as claimed in any one of claims 2 to 11, **characterized in that** the block(s) containing cationic units of the block copolymer is (are) obtained from cationic monomers and the cationic block(s) is (are) preferably homopolymers.

13. The use as claimed in any one of claims 1 and 3 to 12 or the process as claimed in any one of claims 2 to 12, **characterized in that** the organic phase of the emulsion comprises an inner aqueous phase dispersed in said organic phase; the combination of the organic phase and of the inner aqueous phase comprising at least one inner surfactant and/or at least one inner amphiphilic polymer.

14. The use as claimed in any one of claims 1 and 3 to 9 or the process as claimed in any one of claims 2 to 9, **characterized in that** the organic phase of the emulsion comprises an inner organic phase dispersed in said organic phase; the combination of the organic phase and of the inner organic phase comprising at least one inner stabilizing polymer.

15. The use as claimed in any one of claims 1 and 3 to 14 or the process as claimed in any one of claims 2 to 14, **characterized in that** the content of block copolymer represents from 0.1% to 30% by weight relative to the weight of organic phase.

16. The use as claimed in any one of claims 1 and 3 to 15 or the process as claimed in any one of claims 2 to 15, **characterized in that** the block copolymer is added during the preparation of the emulsion of the organic phase and of the aqueous phase; the organic phase optionally comprising a dispersed inner aqueous phase or a dispersed inner organic phase.

17. The use as claimed in any one of claims 1 and 3 to 16 or the process as claimed in any one of claims 2 to 16, **characterized in that** the block copolymer is added once the emulsion of the organic phase and of the aqueous phase has been prepared; the organic phase optionally comprising a dispersed inner aqueous phase or a dispersed inner organic phase.

18. The use as claimed in any one of claims 1 and 3 to 17 or the process as claimed in any one of claims 2 to 17, **characterized in that** the emulsion is a constituent component of a formulation for treating the skin and/or the hair, textiles, for the plant protection sector or the treatment of metal.

19. The use or the process as claimed in claim 18, **characterized in that**:
- the emulsion to be deposited represents a constituent component of an aqueous liquid detergent formulation for washing articles made of textile fiber, comprising at least one anionic surfactant,
- the emulsion is a simple emulsion, the organic phase of which comprises at least one organic or organosilicon hydrophobic active material,
- the emulsion is intended to be deposited on the surface of articles made of textile fiber during a washing operation.

20. The use or the process as claimed in claim 19, **characterized in that** the organic or organosilicon hydrophobic active material is chosen from oils or waxes of plant origin, fatty acid esters, mineral oils, paraffin waxes, nonionic silicone oils or waxes and aminosilicone oils, in particular silicone oils containing hindered piperidyl function(s).

21. The use or the process as claimed in claim 20, **characterized in that** the organic or organosilicon hydrophobic active material in emulsion gives textile surfaces onto which it is deposited creaseproof properties and/or easy-ironing properties and/or antisoiling properties and/or better abrasion resistance.

## Patentansprüche

1. Verwendung von wenigstens einem Blockcopolymer mit wenigstens einem nicht ionischen, hydrophoben Block und wenigstens einem Block aus bei den Verwendungsbedingungen der Emulsion kationischen Einheiten als Hilfsmittel bei der Ablagerung einer Emulsion auf einer Oberfläche; wobei die Emulsion eine organische Phase aufweist, die in einer wässrigen Phase dispergiert ist, und gegebenenfalls wenigstens ein Tensid und/oder wenigstens ein externes amphiphiles Polymer aufweist.

2. Verfahren zur Unterstützung der Ablagerung einer Emulsion auf einer Oberfläche unter Verwendungsbedingungen für die Emulsion durch Hinzufügen zu der Emulsion wenigstens eines Blockcopolymers mit wenigstens einem nicht ionischen, hydrophoben Block und wenigstens einem Block aus bei den Verwendungsbedingungen der Emulsion kationischen Einheiten; wobei die Emulsion eine organische Phase aufweist, die in einer wässrigen Phase dispergiert ist, und gegebenenfalls wenigstens ein Tensid und/oder wenigstens ein externes amphiphiles Polymer aufweist

3. Verwendung gemäß Anspruch 1 oder Verfahren gemäß Anspruch 2, **dadurch gekennzeichnet, dass** der nicht ionische, hydrophobe Block des Blockpolymers erhalten wird aus wenigstens einem Monomer gewählt aus:
- den Estern von Mono- oder Polycarbonsäuren, linear, verzweigt, zyklisch oder aromatisch, die wenigstens einfach ethylenisch ungesättigt sind;
- den Estern von gesättigten Carbonsäuren mit wenigstens 8 bis 30 Kohlenstoffatomen, gegebenenfalls Träger einer Hydroxylgruppe;
- den αβ-ethylenisch ungesättigten Nitrilen, den Vinylethern, den Vinylestern, den vinylaromatischen Monomeren, den Vinyl- oder Vinylidenhalogeniden;
- den Kohlenwasserstoffmonomeren, linear oder verzweigt, aromatisch oder nicht, die wenigstens einfach ethylenisch ungesättigt sind;
- Propylenoxid, Butylenoxid;
einzeln oder als Gemische, sowie die Makromonomere, die von derartigen Monomeren abstammen.

4. Verwendung gemäß Anspruch 1 oder 3 oder Verfahren gemäß Anspruch 2 oder 3, **dadurch gekennzeichnet, dass** der Block aus kationischen Einheiten des Blockcopolymers erhalten wird aus wenigstens einem Monomer gewählt aus:
- den Aminoalkyl(meth)acrylaten, den Aminoalkyl(meth)acrylamiden;
- den Monomeren mit wenigstens einer sekundären, tertiären oder quaternären Aminfunktion oder einer heterozyklischen Gruppe, die ein Stickstoffatom enthält, Vinylamin, Ethylenimin;
- den Diallyldialkylammoniumsalzen;
einzeln oder als Gemische, den entsprechenden Salzen sowie den Makromonomeren, die von derartigen Monomeren abstammen.

5. Verwendung gemäß einem der Ansprüche 1, 3 oder 4 oder Verfahren gemäß einem der Ansprüche 2 bis 4, **dadurch gekennzeichnet, dass** der eine und/oder der andere der beiden Blöcke ein oder mehrere nicht ionische, hydrophile Monomere aufweisen, gewählt aus, einzeln oder als Gemisch oder in Form von Makromonomeren, Ethylenoxid; den Amiden von Mono- oder Polycarbonsäuren, linear, verzweigt, zyklisch oder aromatisch, die wenigstens einfach ethylenisch ungesättigt sind, oder Derivaten; den hydrophilen Estern, die von (Meth)acrylsäure abstammen; den Vinylestern, die es ermöglichen, nach Hydrolyse Polyvinylalkoholblöcke zu erhalten; den Monomeren der Art Zucker.

6. Verwendung gemäß einem der Ansprüche 1 oder 3 bis 5 oder Verfahren gemäß einem der Ansprüche 2 bis 5, **dadurch gekennzeichnet, dass** das Blockcopolymer ein lineares Copolymer mit zwei Blöcken ist.

7. Verwendung gemäß einem der Ansprüche 1 oder 3 bis 5 oder Verfahren gemäß einem der Ansprüche 2 bis 5, **dadurch gekennzeichnet, dass** das Blockcopolymer ein gekämmtes oder ein gepfropftes Copolymer ist mit einem nicht ionischen Skelett, vorzugsweise hydrophil, mit anhängenden kationischen und nicht ionischen hydrophoben Segmenten.

8. Verwendung gemäß einem der Ansprüche 1 oder 3 bis 5 oder Verfahren gemäß einem der Ansprüche 2 bis 5, **dadurch gekennzeichnet, dass** das Blockcopolymer ein Copolymer mit Sternstruktur ist.

9. Verwendung gemäß einem der Ansprüche 1 oder 3 bis 8 oder Verfahren gemäß einem der Ansprüche 2 bis 8, **dadurch gekennzeichnet, dass** die Molmasse in Gewicht des Blockcopolymers zwischen 2000 und 100000 g/mol, vorzugsweise zwischen 2000 und 20000 g/mol liegt.

10. Verwendung gemäß einem der Ansprüche 1 oder 3 bis 9 oder Verfahren gemäß einem der Ansprüche 2 bis 9, **dadurch gekennzeichnet, dass** das Blockcopolymer derart ist, dass die Molmasse in Gewicht der Blöcke aus kationischen Einheiten wenigstens das Zweifache der Masse an nicht ionischen, hydrophoben Blöcken, vorzugsweise wenigstens das Fünffache, beträgt.

11. Verwendung gemäß einem der Ansprüche 1 oder 3 bis 10 oder Verfahren gemäß einem der Ansprüche 2 bis 10, **dadurch gekennzeichnet, dass** der oder die kationischen Blöcke des Blockcopolymers derart sind, dass wenigstens 5 Mol-% der Monomere, aus denen der oder die Blöcke erhalten werden, vorzugsweise wenigstens 30 Mol-%, kationische Monomere sind.

12. Verwendung gemäß einem der Ansprüche 1 oder 3 bis 11 oder Verfahren gemäß einem der Ansprüche 2 bis 11, **dadurch gekennzeichnet, dass** der oder die Blöcke aus kationischen Einheiten des Blockcopolymers aus kationischen Monomeren erhalten werden, und vorzugsweise der oder die kationischen Blöcke Homopolymere sind.

13. Verwendung gemäß einem der Ansprüche 1 oder 3 bis 12 oder Verfahren gemäß einem der Ansprüche 2 bis 12, **dadurch gekennzeichnet, dass** die organische Phase der Emulsion eine interne wässrige Phase aufweist, die in der organischen Phase dispergiert ist; wobei die Gesamtheit aus der organischen Phase und der internen wässrigen Phase wenigstens ein Tensid und/oder wenigstens ein internes amphiphiles Polymer aufweist.

14. Verwendung gemäß einem der Ansprüche 1 oder 3 bis 9 oder Verfahren gemäß einem der Ansprüche 2 bis 9, **dadurch gekennzeichnet, dass** die organische Phase der Emulsion eine interne organische Phase aufweist, die in der organischen Phase dispergiert ist; wobei die Gesamtheit aus der organischen Phase und der internen organischen Phase wenigstens ein internes stabilisierendes Polymer aufweist.

15. Verwendung gemäß einem der Ansprüche 1 oder 3 bis 14 oder Verfahren gemäß einem der Ansprüche 2 bis 14, **dadurch gekennzeichnet, dass** der Gehalt an Blockcopolymer wenigstens 0,1 bis 30 Gew.-% bezogen auf das Gewicht der organischen Phase aufweist.

16. Verwendung gemäß einem der Ansprüche 1 oder 3 bis 15 oder Verfahren gemäß einem der Ansprüche 2 bis 15, **dadurch gekennzeichnet, dass** das Blockcopolymer bei der Zubereitung der Emulsion der organischen Phase und der wässrigen Phase hinzugefügt wird; wobei die organische Phase gegebenenfalls eine dispergierte interne wässrige Phase oder eine dispergierte interne organische Phase aufweist.

17. Verwendung gemäß einem der Ansprüche 1 oder 3 bis 16 oder Verfahren gemäß einem der Ansprüche 2 bis 16, **dadurch gekennzeichnet, dass** das Blockcopolymer hinzugefügt wird, sobald die Zubereitung der Emulsion der organischen Phase und der wässrigen Phase durchgeführt ist; wobei die organische Phase gegebenenfalls eine dispergierte interne wässrige Phase oder eine dispergierte interne organische Phase aufweist.

18. Verwendung gemäß einem der Ansprüche 1 oder 3 bis 17 oder Verfahren gemäß einem der Ansprüche 2 bis 17, **dadurch gekennzeichnet, dass** die Emulsion ein Bestandteil einer Formulierung zur Behandlung der Haut und/oder der Haare, von Textilien, im phytosanitären Bereich, der Behandlung von Metall ist.

19. Verwendung oder Verfahren gemäß Anspruch 18, **dadurch gekennzeichnet, dass**:
- die abzulagernde Emulsion ein Bestandteil einer wässrigen flüssigen Reinigungsformulierung zum Waschen von Waren aus Textilfasern ist, mit wenigstens einem anionischen Tensid,
- die Emulsion eine einfache Emulsion ist, deren organische Phase wenigstens einen hydrophoben organischen oder Organosilizium-Wirkstoff aufweist,
- die Emulsion dazu bestimmt ist, sich während eines Waschvorgangs auf der Oberfläche von Waren aus Textilfasern abzulagern.

20. Verwendung oder Verfahren gemäß Anspruch 19, **dadurch gekennzeichnet, dass** der hydrophobe organische oder Organosilizium-Wirkstoff gewählt wird aus den Ölen oder den Wachsen pflanzlichen Ursprungs, den Fettsäureestern, den Mineralölen, den Paraffinwachsen, den nicht ionischen Silikonölen oder -wachsen, den aminierten Silikonölen, insbesondere den Silikonölen mit blockierten Piperidinylfunktion(en).

21. Verwendung oder Verfahren gemäß Anspruch 20, **dadurch gekennzeichnet, dass** der hydrophobe organische oder Organosilizium-Wirkstoff in Emulsion den Textiloberflächen, auf welchen er abgelagert wird, Antiknittereigenschaften und/oder Bügelhilfsund/oder Antiverschmutzungs- und/oder bessere Abnutzungswiderstandseigenschaften verleiht.
